# EUROPEAN PATENT APPLICATION

(11) **EP 1 547 608 A1**
(43) Date of publication of application: **29.06.2005**
(21) Application number: 03784533.6
(22) Date of filing: 06.08.2003
(51) Int. Cl.: A61K 35/78, A61P 3/04, A61P 3/10, A61P 43/00

(54) **REMEDY**

(30) Priority: 09.08.2002 JP 2002233808; 02.05.2003 JP 2003127518
(71) Applicant: TAKARA BIO INC., Otsu-shi, Shiga 520-2193 (JP)
(72) Inventor: ENOKI, Tatsuji, Kyotanabe-shi, Kyoto 610-0313 (JP); OGAWA, Kinuko, Otsu-shi, Shiga 520-2134 (JP); ONOGI, Hiromu, Kusatsu-shi, Shiga 525-0025 (JP); SUGIYAMA, Katsumi, Otsu-shi, Shiga 520-2134 (JP); MURAKI, Nobuko, Koka-gun, Shiga 529-1851 (JP); SAGAWA, Hiroaki, Kusatsu-shi, Shiga 525-0025 (JP); KATO, Ikunoshin, Koka-shi, Shiga 529-1851 (JP)
(74) Representative: VOSSIUS & PARTNER
(86) International application number: PCT/JP2003/009978
(87) International publication number: WO 2004/014407

(57) **Abstract**

The present invention relates to a therapeutic agent or prophylactic agent for a disease accompanying an abnormality in an amount of insulin or insulin response, an insulin-mimetic action agent, a food, beverage, or feed for treating or preventing a disease accompanying an abnormality in an amount of insulin or insulin response, an agent for enhancement of glucose uptake into a cell, and an agent for induction of an adipocyte differentiation, each comprising as an effective ingredient a processed product derived from a plant belonging to Umbelliferae.

## Description

### TECHNICAL FIELD

The present invention relates to a medicament, food, beverage or feed which is useful for treating or preventing a disease associated with insulin in a living body, for instance, diabetes or obesity.

### BACKGROUND ART

Insulin is a hormone essential for normal metabolism of carbohydrates, proteins and fats in a mammal. Since human suffering from type I diabetes does not sufficiently produce insulin, which is a hormone sustaining life, the administration of insulin from the external is required for survival. Human suffering from type II diabetes is required to be administered with insulin or an agent for promoting insulin secretion in order to control the glucose level in blood from an inappropriate level, due to causations such as deficiency of the amount of insulin produced and insulin resistance, to an appropriate level. However, among humans suffering from type II diabetes, therapeutic effects are not found in some cases even when insulin or the agent for promoting insulin secretion were administered in diabetic patients of which cause is insulin resistance caused by hyperinsulinemia, abnormality in insulin receptor, or abnormality of a downstream signal of the insulin receptor or the like.

In recent years, in order to solve the adverse actions of insulin and the above-mentioned problems, developments have been made on a substance having physiological functions similar to those of insulin (hereinafter referred to as insulin-mimetic substance in some cases). It has been found that a synthetic benzoquinone derivative is an insulin-mimetic substance (see, for instance, WO 99/51225), and that shikonin derived from *Curcuma zedoaeia Roscoe* is an insulin-mimetic substance (see, for instance, Kamei R. and seven others, *Biochem. Biophys. Res. Commun.,* 2002, Vol. 292, P642-651). These insulin-mimetic substances as mentioned above have been expected to ameliorate symptoms by exhibiting physiological activities similar to those of insulin, in not only type I diabetic patients but also type II diabetic patients of which cause is insulin resistance.

*Angelica keiskei* koidz. is a large-scaled perennial plant belonging to Umbelliferae, and a variety of health-promoting effects therefor have been known. For instance, as physiological actions owned by *Angelica keiskei* koidz., prophylactic effect for hypertension, antibacterial action, anti-ulcerative action, suppressive action for gastric acid secretion, anti-cancerous effect, enhancing effect for nerve growth factor production, enhancing action for hepatocyte growth factor production and the like have been known (see, for instance, WO 01/76614). However, the insulin-mimetic action such as anti-diabetic action or anti-obesity action has not so far been known.

*Apium* is a plant belonging to Umbelliferae, and a variety of physiological actions therefor have been known. As the physiological actions of *Apium,* anti-blood coagulating action, carcinostatic action and the like have been known. However, the insulin-mimetic action such as anti-diabetic action or anti-obesity action has not so far been known.

*Petroselium sativum* is a plant belonging to Umbelliferae, and a variety of physiological actions therefor have been known. As the physiological actions of *Petroselium sativum*, amelioration of anemia, prophylactic action for food poisoning, hemostatic action, recovery from fatigue, sweating, diuresis, incubation effects and the like have been known. However, the insulin-mimetic action such as anti-diabetic action or anti-obesity action has not so far been known.

### DISCLOSURE OF INVENTION

An object of the present invention is to develop a processed product derived from a plant being naturally occurring and safe and having an insulin-mimetic action, which is suitable as a food material or medicament material which can be conveniently taken, and to provide a medicament, food, beverage or feed, using the processed product.

Summarizing the present invention hereinbelow, a first invention of the present invention relates to a therapeutic agent or prophylactic agent for a disease accompanying an abnormality in an amount of insulin or insulin response, characterized in that the agent comprises as an effective ingredient a processed product derived from a plant belonging to Umbelliferae.

A second invention of the present invention relates to an agent for an insulin-mimetic action, characterized in that the agent comprises as an effective ingredient a processed product derived from a plant belonging to Umbelliferae.

A third invention of the present invention relates to a food, beverage, or feed for treating or preventing a disease accompanying an abnormality in an amount of insulin or insulin response, characterized in that the agent comprises as an effective ingredient a processed product derived from a plant belonging to Umbelliferae.

A fourth invention of the present invention relates to an agent for enhancement of glucose uptake into a cell, characterized in that the agent comprises as an effective ingredient a processed product derived from a plant belonging to Umbelliferae.

A fifth invention of the present invention relates to an agent for induction of an adipocyte differentiation, characterized in that the agent comprises as an effective ingredient a processed product derived from a plant belonging to Umbelliferae.

In the first to fifth inventions of the present invention, the plant belonging to Umbelliferae is exemplified by, for instance, *Angelica keiskei* koidz., *Apium* or *Petroselium sativum*.

### BRIEF DESCRIPTION OF THE DRAWINGS

Figure 1 is a graph showing the amount of biosynthesized triglyceride of adipocytes which are induced to be differentiated by an extract fraction from root portions of *Angelica keiskei* koidz.
Figure 2 is a graph showing the amount of biosynthesized triglyceride of adipocytes which are induced to be differentiated by the extract fraction-fractionated fraction 3 or 4 from root portions of *Angelica keiskei* koidz.
Figure 3 is a graph showing an enhancing action for glucose uptake by an extract fraction from root portions of *Angelica keiskei* koidz.
Figure 4 is a graph showing the amount of biosynthesized triglyceride of adipocytes which are induced to be differentiated by an extract fraction from leaf portions of *Angelica keiskei* koidz.
Figure 5 is a graph showing the amount of biosynthesized triglyceride of adipocytes which are induced to be differentiated by an extract fraction from leaf portions of *Apium.*
Figure 6 is a graph showing an enhancing action for glucose uptake by an extract fraction from leaf portions of *Apium.*
Figure 7 is a graph showing the amount of biosynthesized triglyceride of adipocytes which are induced to be differentiated by an extract fraction from *Petroselium sativum*.
Figure 8 is a graph showing an enhancing action for glucose uptake by an extract fraction from *Petroselium sativum*.
Figure 9 is a graph showing the amount of biosynthesized triglyceride of adipocytes which are induced to be differentiated by an extract fraction from root portions of *Angelica keiskei* koidz.
Figure 10 is a graph showing an enhancing action for glucose uptake by an extract fraction from root portions of *Angelica keiskei* koidz.
Figure 11 is a graph showing an enhancing action for glucose uptake by an extract fraction from leaf portions of *Angelica keiskei* koidz.
Figure 12 is a graph showing an enhancing action for glucose uptake by an extract fraction-fractionated fraction from root portions of *Angelica keiskei* koidz.
Figure 13 is a graph showing an inhibitory action by cytochalasin B for the enhancing action for glucose uptake by the ethanol extraction fraction from root portions of *Angelica keiskei* koidz.
Figure 14 is a graph showing synergistic effects of the enhancing action for glucose uptake by the ethanol extraction fraction from root portions of *Angelica keiskei* koidz. and insulin.
Figure 15 is a graph showing an enhancing action for glucose uptake by insulin stimulation in the adipocytes which are induced to be differentiated by the ethanol extraction fraction from root portions *of Angelica keiskei* koidz.
Figure 16 is a graph showing the amount of biosynthesized triglyceride of adipocytes which are induced to be differentiated by an extract fraction from stem and leaf portions *of Angelica keiskei* koidz.

### BEST MODE FOR CARRYING OUT THE INVENTION

In the present invention, the plant belonging to Umbelliferae is a plant belonging to Umbelliferae of Angiospermopsida, and exemplified by, for instance, *Angelica keiskei* koidz., *Oenanthe javanica, Cryptotaenia japonica Hassk, Angelica pubescens, Daucus, Apium, Petroselium sativum* and the like. In the present invention, *Angelica keiskei koidz., Apium* and *Petroselium sativum* can be especially suitably used. In the present invention, these can be used alone or in admixture of two or more kinds. In addition, the plant belonging to Umbelliferae usable in the present invention is not particularly limited, and fruit, seed, seed coat, flower, leaf, stem, root, root stem and/or whole plant can be directly used.

The processed product derived from the plant belonging to Umbelliferae usable in the present invention as an effective ingredient is not particularly limited, as long as the processed product has an insulin-mimetic action. The processed product refers to, for instance, an extract, a powder, a squeezed juice, a pulverized product, a chemically processed product, or an enzymatically processed product, and is especially preferably exemplified by an extract, a powder and a squeezed juice. The processed product is not particularly limited as long as the product can be used as the effective ingredient of the present invention. Each of the processed products can be used alone or in admixture of two or more kinds.

Incidentally, in the present invention, the insulin-mimetic action is not particularly limited as long as the action shows physiological activities similar to those of insulin. The insulin-mimetic action is exemplified by metabolic regulatory actions such as enhancement of uptake of a sugar or amino acid in the cell, and synthesis and degradation inhibition of glycogen or protein. The effective ingredient of the present invention may be those at least exhibiting an action for induction of an adipocyte differentiation, or an action for enhancement of glucose uptake into a cell. The presence or absence of the insulin-mimetic action can be conveniently determined in accordance with the method described in Example 3 or 5 set forth below.

In the present invention, the extract refers to a substance obtained through the process of carrying out the extraction procedure with an extraction solvent. The extraction can be carried out as follows by a known extraction method. For instance, the raw material is powdered or cut into thin pieces, and thereafter extracted in a batch process or continuous process using a solvent. The extraction solvent used upon obtaining an extract is not particularly limited, and includes, for instance, hydrophilic or lipophilic solvents such as water, chloroform, alcohols such as ethanol, methanol and isopropyl alcohol, ketones such as acetone and methyl ethyl ketone, methyl acetate, and ethyl acetate, which can be used alone or properly as a mixed solution as desired. The amount of the extraction solvent may be appropriately determined, and the extraction solvent may be used in an amount of preferably from 0.1- to 100-folds that by weight of the raw materials (solid). The extraction temperature may be also appropriately determined according to its purposes. In the case of the water extraction, usually, the extraction temperature is preferably from 4° to 130°C, more preferably from 25° to 100°C. Alternatively, in the case where ethanol is contained in the solvent, the extraction temperature is suitably within the range of from 4° to 60°C. The extraction time may be also determined in consideration of extraction efficiency. It is usually preferable that the raw materials, the extraction solvent and the extraction temperature are set so that the extraction time is preferably from several seconds to several days, more preferably 5 minutes to 24 hours. The extraction procedure may be carried out, for instance, while stirring or allowing the mixture to stand. Also, the extraction procedures may be repeated several times as desired. By the above procedures, an extract derived from the plant belonging to Umbelliferae (hereinafter which may be referred to as the extract of the present invention in some cases) is obtained. The extract is subjected to such a process as filtration, centrifugation, concentration, ultrafiltration or molecular sieving as desired, whereby an extract in which the desired insulin-mimetic substance is concentrated can be prepared. The insulin-mimetic action of the extract or concentrated extract can be conveniently determined in accordance with the method described in Example 3 or 5 set forth below. Alternatively, the plant belonging to Umbelliferae may be processed in the form of tea-leaves by a known method, and an extract using the tea-leaves (for instance, tea of *Angelica keiskei* koidz.) can be used as the extract of the present invention as long as the extract has an insulin-mimetic action. In addition, two or more kinds of these extracts can be contained and used. In the present invention, two or more kinds of extracts obtained by different extraction methods can be contained and used.

In addition, in the present invention, a fraction obtained by fractionating an extract derived from the plant belonging to Umbelliferae by a known method, or a fraction obtained by repeating the fractionation procedures a plural times is also encompassed in the extract of the present invention. The above-mentioned fractionation means include extraction, separation by precipitation, column chromatography, thin-layer chromatography, and the like. The insulin-mimetic substance can also be isolated by further proceeding the purification of the resulting fraction using the insulin-mimetic action as an index.

Alternatively, as a method for preparing a processed product derived from the plant belonging to Umbelliferae from those other than the extract derived from the plant belonging to Umbelliferae, for instance, a plant is dried and powdered, whereby a powder derived from the plant belonging to Umbelliferae can be obtained. It is preferable that the drying is carried out by lyophilization. In addition, the powder may be obtained by freeze-powdering.

In addition, the method for preparing a squeezed juice derived from the plant belonging to Umbelliferae is not particularly limited as long as the method is a known method of squeezing a plant. For instance, squeezing the juice can be accomplished by using a squeezer of a screw-type, a gear-type, a cutter-type or the like, or a juicer. Also, the raw plant may be cut into thin pieces or mashed as a pre-processing, and thereafter squeezed with the above-mentioned juicer or cloth or the like, whereby a squeezed juice can be obtained.

The pulverized product refers to one prepared by pulverizing the plant belonging to Umbelliferae, and its tissue piece is generally larger than the powder. For instance, the pulverized product can be prepared by using a pulverizer. Also, the chemically processed product is not particularly limited, and refers to a product obtained by subjecting a plant belonging to Umbelliferae to an acid processing, an alkali processing, an oxidation processing or a reducing processing. The chemically processed product can be prepared, for instance, by immersing a plant belonging to Umbelliferae in an aqueous solution containing an inorganic acid or organic acid, such as hydrochloric acid, sulfuric acid, nitric acid, citric acid or acetic acid, or an inorganic base or organic base, such as sodium hydroxide, potassium hydroxide or ammonia. The chemically processed product includes all those derived from plants subjected to chemical processing as mentioned above. The enzymatically processed product refers, for instance, to an enzymatically processed product with pectinase, cellulase, xylanase, amylase, mannanase, or glucosidase, an enzymatic reaction product by a microorganism (for instance, a fermented product) or the like. The enzymatically processed product can be prepared, for instance, by allowing the above-mentioned enzyme to act on the plant belonging to Umbelliferae in an appropriate buffer. The enzymatically processed product includes all those derived from plants subjected to the enzymatic processing as mentioned above. Further, the processed product derived from the plant belonging to Umbelliferae encompasses, for instance, juice obtained by cutting stem of the plant belonging to Umbelliferae and obtaining juice from its cross section.

In the present invention, the shape of the processed product derived from the plant belonging to Umbelliferae is not particularly limited as long as the processed product has an insulin-mimetic action, and the processed product may take any form of powder, solid or liquid. In addition, The substance can be used as a processed product derived from the plant belonging to Umbelliferae of the present invention in the form of a granular solid prepared by, for instance, granulating the substance by a known process. The granulation process is not particularly limited, and is exemplified by tumbling granulation, agitation granulation, fluidizing bed granulation, airflow granulation, extruding granulation, compression molding granulation, disintegration granulation, spray granulation, spray-drying granulation or the like. In addition, the processed product can be used as the processed product derived from the plant belonging to Umbelliferae of the present invention in the form of a liquid prepared by, for instance, dissolving a powdery processed product derived from the plant belonging to Umbelliferae in a liquid, for instance, water, an alcohol or the like.

Also, in the present invention, the effective ingredient per se can be used as, for instance, a food, beverage or feed for treating or preventing a disease accompanying an abnormality in the amount of insulin or insulin response described in the present specification. An embodiment of using the effective ingredient per se includes, for instance, a product prepared by forming the extract or powder mentioned above into a tablet. The preparation of the tablet can be carried out in accordance with a known tableting method.

In addition, the present invention provides a food, beverage or feed, comprising a processed product derived from the plant belonging to Umbelliferae in a high concentration or high purity, which is intended to mean that the insulin-mimetic substance is contained in high concentration and/or high purity in the food, beverage or feed of the present invention as compared to conventional foods, beverages or feeds. The food, beverage or feed can be prepared by containing the effective ingredient of the present invention in conventional foods or the like as described later.

Incidentally, in the present invention, the processed product derived from the plant belonging to Umbelliferae may be referred to as the effective ingredient of the present invention, and the therapeutic agent or prophylactic agent for a disease accompanying an abnormality in an amount of insulin or insulin response, comprising the effective ingredient of the present invention may be referred to as the therapeutic agent or prophylactic agent of the present invention in some cases.

No toxicity is especially found in the effective ingredient according to the present invention as mentioned later. Also, there is no risk of the onset of adverse actions. For these reasons, the disease can be safely and appropriately treated and prevented. Therefore, the therapeutic agent, prophylactic agent, food, beverage or feed, each comprising the effective ingredient, is effective for treating or preventing a disease accompanying an abnormality in an amount of insulin or insulin response.

In addition, in the present invention, the disease accompanying an abnormality of an amount of insulin or insulin response include diseases characterized by factors selected from change in insulin level in blood, change in activity level of insulin or an insulin receptor, aberrance in downstream signal of an insulin receptor and combinations thereof. The disease is exemplified by, for instance, diabetes, obesity, arterial sclerosis, cocaine withdrawal symptoms, static cardiac incompetence, cardiovascular seizure, cerebral angiospasm, chromaffinomosa, ganglioneuroblastoma, Huntington's disease, hyperlipemia, and hyperinsulinemia. The diabetes may be exemplified by any of type I diabetes and type II diabetes. In addition, the type II diabetes encompasses a disease of which causation is insulin resistance for which a therapeutic effect is not found even when insulin or a drug for promoting insulin secretion were administered.

There have been known that insulin promotes induction of differentiation of preadipocytes into adipocytes, and that insulin enhances glucose uptake in a matured adipocyte thereby accumulating triglyceride in the adipocyte (Rubin C. S. et al., *J*. *Biol. Chem.,* Vol. 253, No. 20, P7570-7578 (1978)). Specifically, by utilizing this method, an insulin-mimetic action of a test substance can be determined by administering a test substance in place of insulin, and determining an adipocyte differentiation and an amount of biosynthesized triglyceride in the adipocyte.

In addition, there have been known that insulin has an enhancing action for glucose uptake, and that glucose uptake into the cell is enhanced by the action of insulin in a matured adipocyte (Rubin C. S. et al., *J. Biol. Chem.,* Vol. 253, No. 20, P7579-7583 (1978)). Specifically, by utilizing this method, an insulin-mimetic action of a test substance can be determined by administering a test substance in place of insulin, and determining an amount of glucose uptake into a matured adipocyte.

The therapeutic agent or prophylactic agent of the present invention includes ones formed into a preparation by combining the above-mentioned effective ingredient according to the present invention with a known pharmaceutical carrier.

The therapeutic agent or prophylactic agent of the present invention is usually manufactured by formulating the above-mentioned effective ingredient with a pharmacologically acceptable liquid or solid carrier. A solvent, a dispersant, an emulsifier, a buffer, a stabilizer, an excipient, a binder, a disintegrant, a lubricant, or the like is optionally added thereto, so that a solid agent such as a tablet, a granule, a powder, a fine powder, and a capsule, or a liquid agent such as a common liquid agent, a suspension agent or an emulsion agent can be formed. In addition, there can be also made into a dry product which can be made liquid by adding an appropriate liquid carrier before use, or also into an external preparation.

The pharmaceutical carrier can be selected depending upon the administration form and preparation form of the therapeutic agent or prophylactic agent. In the case of an orally administered preparation comprising a solid composition, the preparation can be produced in the form of a tablet, a pill, a capsule, a powder, a fine powder, a granule or the like, and there can be utilized, for instance, starch, lactose, saccharose, mannitol, carboxymethyl cellulose, cornstarch, an inorganic salt or the like. In addition, during the preparation of the orally administered preparation, a binder, a disintegrant, a surfactant, a lubricant, a fluidity accelerator, a flavor, a colorant, a perfume, and the like can be further formulated. In the case of forming into a tablet or pill, for instance, the tablet or pill may be covered with a sugar-coating made of sucrose, gelatin or hydroxypropyl cellulose, or with a film made of a substance soluble in the stomach or intestine as desired. In the case of an orally administered preparation comprising a liquid composition, the preparation can be prepared in the form of a pharmaceutically acceptable emulsion, solution, suspension, syrup, or the like. In this case, for instance, purified water, ethanol or the like is utilized as a carrier. Furthermore, an auxiliary agent such as a wetting agent or a suspending agent, a sweetener, a flavor, an antiseptic, or the like may be added as desired.

On the other hand, in the case of a non-orally administered preparation, the preparation can be prepared by dissolving or suspending the above-mentioned effective ingredient of the present invention in a diluent such as distilled water for injection, physiological saline, an aqueous solution of glucose, vegetable oil for injection, sesame oil, peanut oil, soybean oil, corn oil, propylene glycol or polyethylene glycol, in accordance with a conventional method, and adding a microbicide, a stabilizer, an osmotic regulator, a soothing agent, or the like as desired. It is also possible to produce a solid composition which is dissolved in sterile water or a sterile solvent for injection before use.

The external preparation includes solid, semi-solid or liquid preparations for percutaneous administration or transmucosal (oral or intranasal) administration. The external preparation also includes suppositories and the like. For instance, the external preparation may be prepared as liquid preparations including emulsions, suspensions such as lotions, external tinctures, and liquid agents for transmucosal administration; ointments such as oily ointments and hydrophilic ointments; medical adhesives for percutaneous administration or transmucosal administration such as films, tapes and poultices; and the like.

Each of the above-mentioned various preparations can be appropriately produced in accordance with conventional methods by utilizing known pharmaceutical carriers and the like. Also, the content of the effective ingredient in the preparation is not particularly limited, as long as the content is in an amount so that the effective ingredient can be preferably administered within the dose described below in consideration of administration form, administration method and the like of the preparation.

The therapeutic agent or prophylactic agent of the present invention is administered via an administration route appropriate for each of the preparation form. The administration method is also not limited to specific one. The agent can be administered internally, externally (or topically) or by injection. The injection can be administered, for instance, intravenously, intramuscularly, subcutaneously, intracutaneously, or the like. As to an external preparation, for instance, a suppository may be administered according to its proper administration method.

The dose of the therapeutic agent or prophylactic agent of the present invention is changeable and properly set depending upon its preparation form, administration method, purpose of use, and age, body weight, symptom or the like of a patient to which the therapeutic agent or prophylactic agent is applied, or the like. Generally, the dose of the agent, in terms of the dose of the above-mentioned effective ingredient contained in the preparation, is preferably from 0.1 µg to 1 g/kg weight for human (for instance, adult) per day. As a matter of course, the dose varies depending upon various conditions, so that an amount smaller than the dose mentioned above may be sufficient, or an amount exceeding the dose range may be required. Administration may be carried out once or in several divided portions in a day within the desired dose range. Also, the therapeutic agent or prophylactic agent of the present invention can be directly orally administered, or the agent can be added to any foodstuffs to be taken on a daily basis. In addition, by using the effective ingredient of the present invention together with a substance having an action equivalent to the effective ingredient of the present invention, for instance, insulin, synergistic effects of these substances can be expected as described in Example 20.

In addition, the present invention can provide an insulin-mimetic action agent comprising the above-mentioned effective ingredient. The insulin-mimetic action agent may be the above-mentioned effective ingredient itself, or a composition comprising the above-mentioned effective ingredient. The insulin-mimetic action agent may be prepared by, for instance, formulating the above-mentioned effective ingredient with other ingredients (for instance, insulin or the like) which can be used for the same application as the effective ingredient, and forming into a form of reagent usually used according to the above-mentioned process for preparing the therapeutic agent or prophylactic agent. The content of the above-mentioned effective ingredient in the insulin-mimetic action agent is not particularly limited, as long as the content is in an amount so that the desired effects of the present invention can be exhibited in consideration of administration method, purpose of use or the like of the insulin-mimetic action agent. The content of the effective ingredient is, for instance, from 0.01 to 100% by weight. Also, the amount of the insulin-mimetic action agent used is not particularly limited, as long as the desired effects of the present invention can be exhibited. Especially in the case where the insulin-mimetic action agent is administered to a living body, the insulin-mimetic action agent may be preferably used in an amount so that the effective ingredient can be administered within the dose range of the effective ingredient for the above-mentioned therapeutic agent or prophylactic agent. The insulin-mimetic action agent is useful in a disease accompanying an abnormality of an amount of insulin or insulin response. Also, the insulin-mimetic action agent can be used for the manufacture of a food, beverage or feed for treating or preventing these diseases. The food, beverage, or feed can be used according to the above-mentioned food, beverage or feed for treating or preventing a disease accompanying an abnormality in an amount of insulin or insulin response. In addition, the insulin-mimetic action agent is also useful for screening of drugs for diseases accompanying an abnormality in an amount of insulin or insulin response. Furthermore, the insulin-mimetic action agent is useful for studies on mechanisms of an action on cells by insulin, or functional studies relating to physical changes in the cells.

In addition, the amount of insulin in blood can be expected to be lowered by administering the insulin-mimetic action agent of the present invention to human. In other words, the insulin-mimetic action agent of the present invention can also be used as a therapeutic or prophylactic agent for a disease requiring the lowering of the amount of insulin for the treatment or prevention. The disease is not particularly limited, and is exemplified by hyperinsulinemia, Alzheimer's disease and the like. In addition, since reports have been made that the stimulation via an insulin receptor and the effect of extended longevity are closely related (*Science,* vol. 299, P572-574 (2003); *Nature*, vol. 424, P277-284 (2003)), the insulin-mimetic action agent of the present invention can also be used as an agent for anti-aging.

No toxicity is especially found in the effective ingredient according to the present invention as described later. Also, there is no risk of the onset of adverse actions. For these reasons, the insulin-mimetic action can be safely and appropriately exhibited. Therefore, the medicament, food, beverage or feed of the present invention comprising the effective ingredient is effective for treating or preventing a disease accompanying an abnormality of an amount of insulin or insulin response.

In addition, the present invention provides a food, beverage or feed for treating or preventing a disease accompanying an abnormality of an amount of insulin or insulin response in which the above-mentioned effective ingredient is contained, added and/or diluted. Since the food, beverage or feed of the present invention has an insulin-mimetic action, the food, beverage or feed is very useful in amelioration of symptoms or prevention for a disease accompanying an abnormality of an amount of insulin or insulin response. Furthermore, the food or beverage of the present invention is a food or beverage for lowering blood sugar level, having the action of lowering a blood sugar level, so that the food or beverage is useful as a functional food or beverage effective for an individual who cares for one's blood sugar level or an individual who cares for one's body fat.

As used herein, the term "containing(ed)" refers to an embodiment of containing the effective ingredient usable in the present invention in the food, beverage or feed; the term "adding(ed)" refers to an embodiment of adding the effective ingredient usable in the present invention to a raw material for the food, beverage or feed; and the term "diluting(ed)" refers to an embodiment of adding a raw material for the food, beverage or feed to the effective ingredient usable in the present invention.

The process for preparing the food, beverage or feed of the present invention is not particularly limited. For instance, formulation, cooking, processing, and the like can be carried out in accordance with those generally employed for foods, beverages or feeds, and the food, beverage or feed of the present invention can be prepared by the general methods for preparing a food, beverage or feed, as long as the resulting food, beverage or feed may contain the above-mentioned effective ingredient of the present invention, wherein the effective ingredient has insulin-mimetic action.

The food or beverage of the present invention is not particularly limited. The food or beverage includes, for instance, processed agricultural and forest products, processed stock raising products, processed marine products and the like, including processed grain products such as processed wheat products, processed starch products, processed premix products, noodles, macaronis, bread, bean jam, buckwheat noodles, wheat-gluten bread, rice noodle, *fen-tiao*, and packed rice cake; processed fat and oil products such as plastic fat and oil, tempura oil, salad oil, mayonnaise, and dressing; processed soybean products such as tofu products, soybean paste, and fermented soybeans; processed meat products such as ham, bacon, pressed ham, and sausage; marine products such as frozen ground fish, boiled fish paste, tubular roll of boiled fish paste, cake of ground fish, deep-fried patty of fish paste, fish ball, sinew, fish meat ham, sausage, dried bonito, products of processed fish egg, marine cans, and preserved food boiled down in soy sauce (*tsukudani*); milk products such as raw material milk, cream, yogurt, butter, cheese, condensed milk, powder milk, and ice cream; processed vegetable and fruit products such as paste, jam, pickled vegetables, fruit beverages, vegetable beverages, and mixed beverages; confectionaries such as chocolates, biscuits, sweet bun, cake, rice cake snacks and rice snacks; alcohol beverages such as *sake*, Chinese liquor, wine, whiskey, Japanese distilled liquor (*shochu*), vodka, brandy, gin, rum, beer, refreshing alcoholic beverages, fruit liquor, and liqueur; luxury drinks such as green tea, tea, oolong tea, coffee, refreshing beverages and lactic acid beverages; seasonings such as soy sauce, sauce, vinegar, and sweet rice wine; canned, binned or pouched foods such as rice topped cooked beef and vegetable, rice boiled together with meat and vegetables in a small pot, steamed rice with red beans, curry roux and rice, and other precooked foods; semi-dry or concentrated foods such as liver pastes and other spreads, soups for buckwheat noodles or wheat noodles, and concentrated soups; dry foods such as instant noodles, instant curry roux, instant coffee, powder juice, powder soup, instant soybean paste (*miso*) soup, precooked foods, precooked beverages, and precooked soup; frozen foods such as *sukiyaki*, pot-steamed hotchpotch, split and grilled eel, hamburger steak, *shao-mai*, dumpling stuffed with minced pork, various sticks, and fruit cocktails; solid foods; liquid foods (soups); spices; and the like, in which each of the foods and beverages comprises the above-mentioned ingredient according to the present invention.

In the food or beverage of the present invention, the above-mentioned effective ingredient is contained, added and/or diluted, alone or in plurality, and its shape is not particularly limited, as long as the effective ingredient is contained in an amount necessary for exhibiting its insulin-mimetic action. For instance, the shape includes those which can be taken orally such as tablets, granules and capsules.

In addition, as to the beverage of the present invention, there can be prepared into healthcare drink by mixing the effective ingredient of the present with a squeezed juice of a plant other than those belonging to Umbelliferae, for instance, a vegetable, a fruit or the like, or squeezing the plant together with the plant belonging to Umbelliferae. For instance, the healthcare drink having insulin-mimetic action can be prepared by diluting a squeezed juice of *Angelica keiskei* koidz. with water, or mixing the squeezed juice with a squeezed juice of *Daucus, Brassica Rapa* var. *pervidis* (*komatsuna*), Japanese turnip, Qing gin cai, tomato, mandarin orange, lemon, grapefruit, kiwi, spinach, radish, Japanese radish (*daikon*), Chinese cabbage, cabbage, sunny lettuce, lettuce, *Allium odorum,* okra, green pepper, cucumber, kidney beans, green soybeans, pea, Indian corn, garlic, Rocket, loquat, *Citrus natsudaidai*, *amanatsu*, or the like, cow's milk, soybean milk or the like.

The content of the above-mentioned effective ingredient in the food or beverage of the present invention is not particularly limited, and the content can be appropriately selected from the viewpoints of sensory aspect and exhibition of activity. The content of the effective ingredient is, for instance, preferably 0.00001% by weight or more, more preferably from 0.0001 to 10% by weight, even more preferably from 0.0006 to 6% by weight, per 100% by weight of the food. The content is, for instance, preferably 0.00001% by weight or more, more preferably from 0.0001 to 10% by weight, even more preferably from 0.0006 to 6% by weight, per 100% by weight of the beverage. Also, it is preferable that the food or beverage of the present invention is taken so that the effective ingredient contained therein is taken in an amount of from 0.001 mg to 10 g/kg, preferably from 0.1 mg to 1 g/kg, per day for human (for instance, adult).

Also, as mentioned above, when the effective ingredient is provided as a food or the like in the form of a tablet, the content of the effective ingredient is, for instance, from 0.01 to 100% by weight. On the other hand, when the squeezed juice is directly used as a beverage, the content of the effective ingredient of the present invention is, for instance, from 0.01 to 100% by weight.

In addition, the present invention provides a feed for an organism having insulin-mimetic action, prepared by containing, adding and/or diluting the above-mentioned effective ingredient. In still another embodiment, the present invention also provides a method of feeding an organism, characterized by administering the above-mentioned effective ingredient to the organism. In still yet another embodiment, the present invention provides an organism feeding agent characterized in that the organism feeding agent comprises the above-mentioned effective ingredient.

In these inventions, the organisms are, for instance, culturing or breeding animals, pet animals, and the like. The culturing or breeding animal is exemplified by cattle, laboratory animals, poultry, pisces, crustaceae or shellfish. The feed is exemplified by a feed for sustenance of and/or amelioration in physical conditioning. The organism feeding agent is exemplified by immersion agents, feed additives, and beverage additives.

According to these inventions, the same effects can be expected to be exhibited as those of the above-mentioned therapeutic agent or prophylactic agent of the present invention, on the basis of the insulin-mimetic action of the above-mentioned effective ingredient usable in the present invention, in the organism exemplified above for applying these. In other words, the above-mentioned feed or the like has a therapeutic or prophylactic effect for a disease accompanying an abnormality in an amount of insulin or insulin response in the organism.

The above-mentioned effective ingredient usable in the present invention is usually administered in an amount of preferably from 0.01 mg to 2000 mg, per day per 1 kg of the body weight of the subject organism. The administration can be made by previously adding and mixing the effective ingredient of the present invention in a raw material for an artificially formulated feed to be given to a subject organism, or mixing the effective ingredient of the present invention with a powder raw material for an artificially formulated feed, and thereafter further adding and mixing the mixture with other raw materials. The content of the above-mentioned effective ingredient in the feed is not particularly limited. The content can be appropriately set in accordance with its purposes, and the content is in a ratio of preferably from 0.001 to 15% by weight.

The process for preparing the feed according to the present invention is not particularly limited, and its composition may be set in accordance with a general feed, as long as the above-mentioned effective ingredient according to the present invention having insulin-mimetic action may be contained in the feed prepared.

The organism to which the present invention can be applied is not limited. The culturing or breeding animals include cattle such as *Equus, Bos, Porcus, Ovis, Capra, Camelus,* and *Lama*; experimental animals such as mice, rats, guinea pigs, and rabbits; poultry such as *Chrysolophus,* ducks, *Meleagris,* and *Struthioniformes;* and the pet animals includes dogs, cats, and the like, so that the feed can be widely applied.

By allowing a subject organism to take the feed comprising the above-mentioned effective ingredient usable in the present invention having insulin-mimetic action, or immersing a subject organism into a solution containing the above-mentioned effective ingredient usable in the present invention having insulin-mimetic action, the physical conditions of the cattle, experimental animals, poultry, pet animals or the like can be well sustained or ameliorated. These examples are encompassed in the feeding method of the present invention.

In addition, the present invention can provide an agent for enhancement of glucose uptake into a cell comprising the above-mentioned effective ingredient. The agent for enhancement of glucose uptake may be the above-mentioned effective ingredient itself, or a composition comprising the above-mentioned effective ingredient. The agent for enhancement of glucose uptake may be prepared by, for instance, formulating the above-mentioned effective ingredient with other ingredients (for instance, insulin or the like) which can be used for the same application as the effective ingredient, and forming into a form of reagent usually used according to the above-mentioned process for preparing the therapeutic agent or prophylactic agent. The content of the above-mentioned effective ingredient in the agent for enhancement of glucose uptake is not particularly limited, as long as the content is in an amount so that the desired effects of the present invention can be exhibited in consideration of administration method, purpose of use or the like of the agent for enhancement of glucose uptake. The content of the effective ingredient is, for instance, from 0.01 to 100% by weight. Also, the amount of the agent for enhancement of glucose uptake used is not particularly limited, as long as the desired effects of the present invention can be exhibited. Especially in the case where the agent for enhancement of glucose uptake is administered to a living body, the agent for enhancement of glucose uptake may be preferably used in an amount so that the effective ingredient can be administered within the dose range of the effective ingredient for the above-mentioned therapeutic agent or prophylactic agent. The agent for enhancement of glucose uptake is useful in a disease requiring an enhancing action for glucose uptake into a cell for the treatment or prevention. The disease is exemplified by, for instance, the above-mentioned disease accompanying an abnormality in an amount of insulin or insulin response, as well as cardiac diseases, especially cardiac infarction and post-ischemic injury of the heart, and the like. In addition, since the agent for enhancement of glucose intake enhances glucose uptake by a cell, the action is exhibited in a muscle cell, whereby an action for enhancing muscles or an action for recovery from fatigue can be induced. Also, the agent for enhancement of glucose uptake can be used for the manufacture of a food, beverage or feed for treating or preventing these diseases. The food, beverage, or feed can be used according to the above-mentioned food, beverage or feed for treating or preventing a disease accompanying an abnormality in an amount of insulin or insulin response. In addition, the agent for enhancement of glucose uptake is also useful for screening of drugs for diseases requiring an enhancing action for glucose uptake into a cell for the treatment or prevention. Furthermore, the agent for enhancement of glucose uptake is useful for studies on mechanisms of action for glucose uptake by the cell, or functional studies on physical changes in the cells and the like.

In addition, the present invention can provide an agent for induction of an adipocyte differentiation comprising the above-mentioned effective ingredient. The precursor cell that can be induced to be subjected to adipocyte differentiation by the agent for induction of differentiation is not particularly limited, as long as the cell is capable of differentiating into adipocytes. The precursor cell includes, preadipocyte, fibroblast, mesenchymal stem cell and the like. The agent for induction of differentiation may be the above-mentioned effective ingredient itself, or a composition comprising the above-mentioned effective ingredient. The agent for induction of differentiation may be prepared by, for instance, formulating the above-mentioned effective ingredient with other ingredients (for instance, insulin or the like) which can be used for the same application as the effective ingredient, and forming into a form of reagent usually used according to the above-mentioned process for preparing the therapeutic agent or prophylactic agent. The content of the above-mentioned effective ingredient in the agent for induction of differentiation is not particularly limited, as long as the content is in an amount so that the desired effects of the present invention can be exhibited in consideration of administration method, method of use or the like of the agent for induction of differentiation. The content of the effective ingredient is, for instance, from 0.01 to 100% by weight. Also, the amount of the agent for induction of differentiation used is not particularly limited, as long as the desired effects of the present invention can be exhibited. Especially in the case where the agent for induction of differentiation is administered to a living body, the agent for induction of differentiation may be preferably used in an amount so that the effective ingredient can be administered within the dose range of the effective ingredient for the above-mentioned therapeutic agent or prophylactic agent. The agent for induction of differentiation is useful in a disease requiring an action for induction of an adipocyte differentiation for the treatment or prevention. The disease is exemplified by, for instance, the above-mentioned disease accompanying an abnormality in an amount of insulin or insulin response, as well as gout, fatty liver, cholecystolithiasis, emmeniopathy, infertility, and the like. The agent for induction of differentiation can be used for the manufacture of a food, beverage or feed for treating or preventing these diseases. The food, beverage, or feed can be used according to the above-mentioned food, beverage or feed for treating or preventing a disease accompanying an abnormality in an amount of insulin or insulin response. In addition, the agent for induction of differentiation is also useful for screening of drugs for diseases requiring an action for induction of an adipocyte differentiation for the treatment or prevention. Furthermore, the agent for induction of differentiation is useful for studies on mechanisms of action for induction of an adipocyte differentiation, or functional studies on physical changes in the cells and the like.

No toxicity is found even when the above-mentioned effective ingredient usable in the present invention is administered in an amount effective for the exhibition of its action. For instance, in the case of oral administration, no cases of deaths are found even when each of an extract from *Angelica keiskei* koidz., *Apium* or *Petroselium sativum* is administered to a mouse at 1 g/kg in a single dose. In addition, no cases of deaths are found even when the above-mentioned effective ingredient is orally administered to a rat at 1 g/kg in a single dose.

### EXAMPLES

The present invention will be described more concretely hereinbelow by means of the examples, but the present invention is by no means limited to these descriptions. Unless specified otherwise, "%" in the examples means "% by volume."

### Example 1 Preparation of Extract Fraction from Root Portions of Angelica keiskei Koidz.

(1) One-hundred milliliters of chloroform was added to 10 g of a powder prepared by lyophilizing root portions of *Angelica keiskei* koidz. and pulverizing the lyophilized product, and extracted at room temperature for 30 minutes. After suction filtration, the same procedures were repeated for the residue. These chloroform extracts were combined, and the combined extract was concentrated under reduced pressure with a rotary evaporator. Thereafter, the dry solid was dissolved in 2.5 mL of dimethyl sulfoxide, to give a chloroform extract fraction from root portions of *Angelica keiskei* koidz.
(2) One-hundred milliliters of ethanol was added to the residue after the chloroform extraction of item (1) of Example 1, and extracted at room temperature for 30 minutes. After suction filtration, the same procedures were repeated for the residue. The ethanol extracts were combined, and the combined extract was concentrated under reduced pressure with a rotary evaporator. Thereafter, the dry solid was dissolved in 2.5 mL of dimethyl sulfoxide, to give an ethanol extract fraction from root portions *of Angelica keiskei* koidz.

### Example 2 Fractionation of Extract Fraction from Root Portions of Angelica keiskei Koidz.

Twenty-four liters of ethyl acetate was added to 5.8 kg of dry powder of root portions of *Angelica keiskei* koidz., and extracted at room temperature for 3 hours. After suction filtration, the mixture was separated into an ethyl acetate extract and a residue. Two-hundred milliliters of the resulting ethyl acetate extract was concentrated under reduced pressure with a rotary evaporator. Thereafter, the concentrate was dissolved in chloroform, and fractionated with silica chromatography. The conditions therefor are given below. BW-300SP (manufactured by Fuji Silysia Chemical Ltd.: 100 mL) was used as silica gel. The elution was carried out sequentially with chloroform (500 mL), chloroform : methanol (ratio by volume, hereinafter the same) = 100 : 1 (300 mL), ethyl acetate (200 mL). The eluates were fractionated and collected in the order of Fraction 1 (280 mL), Fraction 2 (200 mL), Fraction 3 (280 mL) and Fraction 4 (240 mL). Each of the fractions was concentrated under reduced pressure to dryness, and thereafter the residue was dissolved in 2 mL of ethanol, to give silica column-fractionated fractions 1 to 4.

### Example 3 Induction of Adipocyte Differentiation by Extract Fraction from Root Portions of Angelica keiskei Koidz.

(1) Induction of Adipocyte Differentiation
   The induction of an adipocyte differentiation was carried out by partially modifying the above-mentioned method of Rubin C. S. et al. Preadipocyte cell line 3T3-L1 (ATCC CCL-92.1) was suspended in a 10% bovine fetal serum (manufactured by GIBCO)-containing Dulbecco's modified Eagle's medium (manufactured by Sigma, D6046) containing 200 µM ascorbic acid (hereinafter referred to as A-D-MEM) so as to have a concentration of 4 × 10³ cells/mL, and the suspension was put in each well of a 12-well microtiter plate in an amount of 2 mL per well. The cells were cultured at 37°C for 7 days in the presence of 5% carbon dioxide gas. The medium was exchanged with the same medium on the second day and the fourth day. On the seventh day, the medium was exchanged with A-D-MEM containing 0.25 µM dexamethasone, and thereafter the chloroform extract fraction from root portions of *Angelica keiskei* koidz. prepared in item (1) of Example 1 or the ethanol extract fraction from root portions of *Angelica keiskei* koidz. prepared in item (2) of Example 1 was added thereto so as to have a final concentration of 0.1%. Here, there were set a group with addition of 4 µl of an aqueous solution containing 5 mg/mL insulin (manufactured by TAKARA BIO INC.) as a positive control, and a group with addition of water as a negative control. The medium was exchanged with A-D-MEM at a point 45 hours after the addition of each component. Four microliters of the chloroform extract fraction from root portions of *Angelica keiskei* koidz. or the ethanol extract fraction from *Angelica keiskei* koidz., 2 µl of an aqueous solution containing 5 mg/mL insulin as a positive control or water as a negative control was put to each well. The cells were cultured for additional 7 days. The medium was exchanged on the second day and the fourth day, when 4 µl of the chloroform extract fraction from root portions of *Angelica keiskei* koidz. or the ethanol extract fraction from root portions of *Angelica keiskei* koidz., or 2 µl of an aqueous solution containing 5 mg/mL insulin as a positive control, or water as a negative control was put to each well.
(2) Determination of Amount of Biosynthesized Triglyceride
   The amount of biosynthesized triglyceride in the adipocytes was determined as an index of induction of differentiation into matured adipocytes, and as evaluation of insulin-mimetic action. After the termination of the culture, the medium was removed, and the cells were washed twice with a phosphate buffer. One milliliter of a solvent of hexane : isopropanol = 3 : 2 was added thereto. The mixture was allowed to stand at room temperature for 30 minutes, and thereafter the supernatant was collected. The procedures were repeated again, and 2 mL of the resulting supernatant was concentrated to dryness. The precipitate was dissolved in 100 µl of isopropanol, and thereafter the amount of triglyceride contained in 10 µl of the resulting solution was determined with Triglyceride G-Test (manufactured by Wako Pure Chemical Industries, Ltd., code 276-69801). All of the determinations were carried out twice.

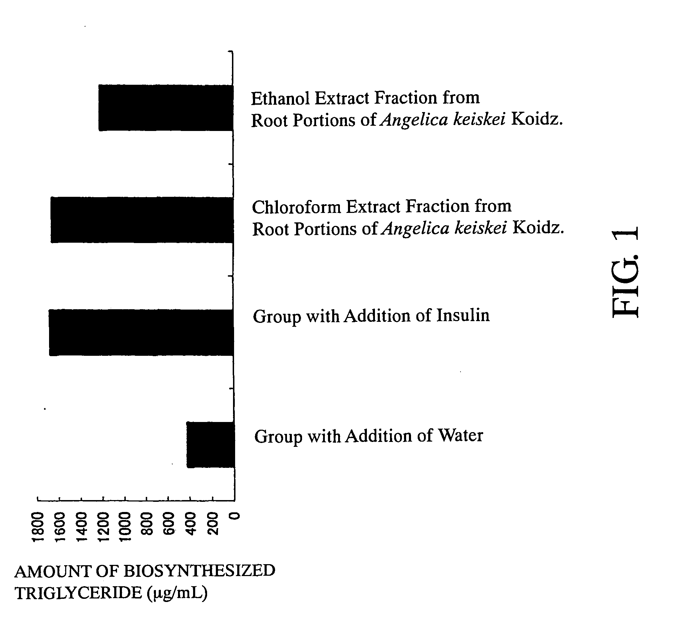

As a result, the induction of triglyceride biosynthesis could be confirmed in the group with addition of the chloroform extract fraction from root portions of *Angelica keiskei* koidz. or the ethanol extract fraction from root portions of *Angelica keiskei* koidz., as compared to the group with addition of water, in the same manner as that in the group with addition of insulin. In other words, the chloroform extract fraction from root portions of *Angelica keiskei* koidz. and the ethanol extract fraction from root portions of *Angelica keiskei* koidz. were found to have the actions of induction of an adipocyte differentiation. The results are shown in Figure 1. In Figure 1, the axis of abscissas is each sample, and the axis of ordinates is the amount of biosynthesized triglyceride (µg/mL).

### Example 4 Induction of Adipocyte Differentiation by Fractionated Fraction of Extracts from Root Portions of Angelica keiskei Koidz.

The activity of induction of an adipocyte differentiation of each fraction of the extract fraction from root portions of *Angelica keiskei* koidz. prepared in Example 2 was assayed in the same manner as in the method described in Example 3. A 2.875 mg/mL silica column-fractionated fraction 3 or a 10.825 mg/mL silica column-fractionated fraction 4 was added as a sample in an amount of 4 µl each. Here, there were set a group with addition of 4 µl of an aqueous solution containing 5 mg/mL insulin as a positive control and a group with addition of water as a negative control. Thereafter, the medium and the sample were exchanged in the same manner as in the method described in Example 3. The amount of triglyceride in the adipocytes was determined 7 days after addition of the sample.

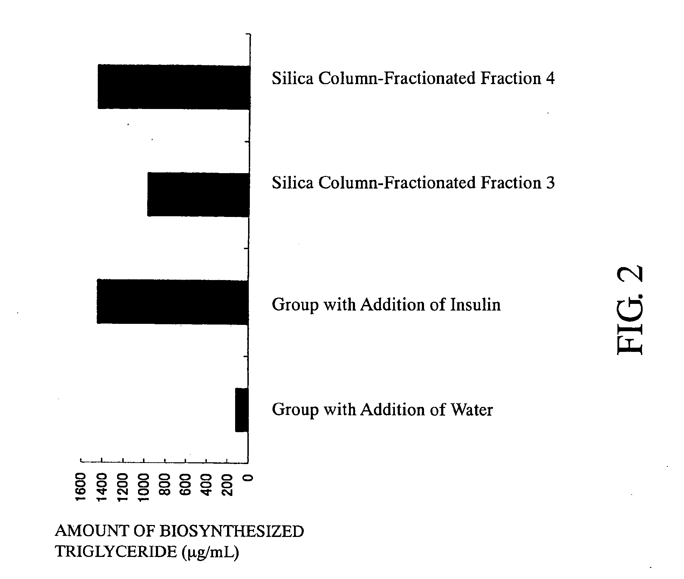

As a result, the induction of triglyceride biosynthesis could be confirmed in the group with addition of the silica column-fractionated fraction 3 and the group with addition of the silica column-fractionated fraction 4, as compared to the group with addition of water, in the same manner as that in the group with addition of insulin. In other words, the silica column-fractionated fraction 3 and the silica column-fractionated fraction 4 were found to have the actions of induction of an adipocyte differentiation. The results are shown in Figure 2. In Figure 2, the axis of abscissas is each sample, and the axis of ordinates is the amount of biosynthesized triglyceride (µg/mL).

### Example 5 Enhancing Action for Glucose Uptake of Extract Fraction from Root Portions of Angelica keiskei Koidz.

(1) Preparation of Matured Adipocytes
   The induction of differentiation into matured adipocytes was carried out by partially modifying the above-mentioned method of Rubin C. S. et al. 3T3-L1 cells (ATCC CCL-92.1) were suspended in a 10% bovine fetal serum (manufactured by GIBCO)-containing Dulbecco's modified Eagle's medium (manufactured by Sigma, D6046) containing 200 µM ascorbic acid so as to have a concentration of 4 × 10³ cells/mL, and the suspension was put in each well of a 12-well microtiter plate in an amount of 2 mL per well. The cells were cultured at 37°C for 7 days in the presence of 5% carbon dioxide gas. On the seventh day, the medium was exchanged with 2 mL of a 10% bovine fetal serum (manufactured by GIBCO)-containing Dulbecco's modified Eagle's medium containing 200 µM ascorbic acid, 0.25 µM dexamethasone, 10 µg/mL insulin (manufactured by TAKARA BIO Inc.), and 0.5 mM 3-isobutyl 1-methylated xanthine (manufactured by nacalai tesque, 19624-44). After 45 hours, the medium was exchanged with 2 mL of a 10% bovine fetal serum-containing Dulbecco's modified Eagle's medium containing 200 µM ascorbic acid and 5 µg/mL insulin. The medium was further exchanged on after 2 days and after 4 days, and the cells were cultured for 7 days, to give matured adipocytes.
(2) Determination of Enhancing Action for Glucose Uptake into Matured Adipocytes
   As the evaluation of the enhancing action for glucose uptake, and also as the evaluation of the insulin-mimetic action, the amount of 2-deoxyglucose uptake into matured adipocytes during the stimulation of the samples in the adipocytes was determined.

After the termination of the culture, the medium was removed, and the cells were washed twice with a 0.1 w/v% bovine serum albumin (manufactured by Sigma, A8022)-containing Dulbecco's modified Eagle's medium. Thereafter, 1 mL of the same medium containing a dimethyl sulfoxide solution of the chloroform extract fraction from root portions of *Angelica keiskei* koidz. or a dimethyl sulfoxide solution of the ethanol extract fraction from root portions of *Angelica keiskei* koidz. was put to each cell to have a final concentration of 0.025% or 0.008%, respectively. The cells were cultured overnight at 37°C in the presence of 5% carbon dioxide gas. A group with addition of no sample was set as a negative control. After the overnight culture, the cells were washed twice with HEPES buffer (140 mM NaCl, 5 mM KCI, 2.5 mM MgSO₄, 1 mM CaCl₂, 20 mM HEPES-Na (pH 7.4)). The amount 0.9 mL of the same buffer containing a dimethyl sulfoxide solution of the chloroform extract fraction from root portions of *Angelica keiskei* koidz. or a dimethyl sulfoxide solution of the ethanol extract fraction from root portions of *Angelica keiskei* koidz. was put to each well to have a final concentration of 0.025% or 0.008%, respectively. The cells were cultured at 37°C for 75 minutes. During the culture, as a positive control, there was set a group with addition of insulin to the well without the addition of sample after 45 minutes passed, so as to have a final concentration of 1 µg/mL. Thereafter, 100 µL of HEPES buffer containing 0.5 µCi/mL 2-deoxy-[1,2-³H(N)]-glucose (manufactured by PerkinElmer Life Sciences, Inc., NET549A) and 1 mM 2-deoxyglucose (manufactured by nacalai tesque, 10722-11) was added thereto. The cells were further cultured at 37°C for 10 minutes. After the termination of the culture, the supernatant was removed, and the cells were washed 3 times with a phosphate buffer cooled to 4°C. Thereafter, 0.5 mL of a 1%-Nonidet P-40-containing phosphate buffer was added thereto to lyse the cells, whereby 2-deoxy-[1,2-³H(N)]-glucose subjected to uptake into the cells was eluted. The radioactivity was determined with a liquid scintillation counter LS6500 (manufactured by Beckman Coulter, Inc.) using 25 µl of the supernatant with Aquasol-2 (manufactured by PerkinElmer Life Sciences, Inc., 6NE9529) as a scintillation cocktail.

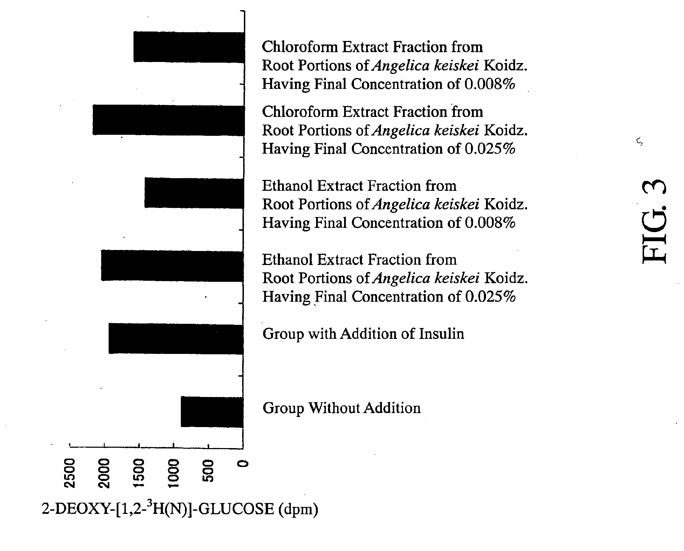

As a result, the enhancement of 2-deoxy[1,2-³H(N)]-glucose uptake was found in the group with addition of the chloroform extract fraction from root portions of *Angelica keiskei* koidz. and the group with addition of the ethanol extract fraction from root portions of *Angelica keiskei* koidz. at each concentration, as compared to the negative control, in the same manner as that in the group with addition of insulin. In other words, the chloroform extract fraction from root portions of *Angelica keiskei* koidz. and the ethanol extract fraction from root portion of *Angelica keiskei* koidz. were found to show the enhancing activity for glucose uptake. The results are shown in Figure 3. In Figure 3, the axis of abscissas is each sample, and the axis of ordinates is the amount of 2-deoxy[1,2-³H(N)]-glucose (dpm).

### Example 6 Preparation of Extract Fraction from Leaf Portions of Angelica keiskei Koidz.

Forty milliliters of distilled water, ethanol or ethyl acetate was added to each 2 g of dry powder of leaf portions of *Angelica keiskei* koidz., and extracted for 30 minutes. The mixture was separated into the extract and the residue by centrifugation. Next, the procedure of extracting the residue with 30 mL of each solvent was repeated twice. Here, the extraction with water was carried out at 60°C, and the extraction with ethanol and the extraction with ethyl acetate were carried out at room temperature. The resulting extracts were collected, and the combined extract was concentrated with a rotary evaporator. Finally, the water extract was dissolved in 10 mL of distilled water, to give a water extract fraction from leaf portions of *Angelica keiskei* koidz. The ethanol extract was dissolved in 8 mL of dimethyl sulfoxide, to give an ethanol extract fraction from leaf portions of *Angelica keiskei* koidz. The ethyl acetate extract was dissolved in 5 mL of dimethyl sulfoxide, to give an ethyl acetate extract fraction from leaf portions of *Angelica keiskei* koidz.

### Example 7 Induction of Adipocyte Differentiation by Extract Fraction from Leaf Portions of Angelica keiskei Koidz.

The action of induction of differentiation (insulin-mimetic activity) of the water extract fraction from leaf portions of *Angelica keiskei* koidz. and the ethanol extract fraction from leaf portions of *Angelica keiskei* koidz. prepared in Example 6 into matured adipocytes was determined in the same manner as in the method of Example 3.

Specifically, as a sample, there was set a group with addition of an aqueous solution of the water extract fraction from leaf portions of *Angelica keiskei* koidz. to each well to have a final concentration of 0.4%, 0.2%, or 0.1%, respectively, or a group with addition of a dimethyl sulfoxide solution of ethanol extract fraction from leaf portions of *Angelica keiskei* koidz. to each well to have a final concentration of 0.066% or 0.022%, respectively. Here, a group with addition of 4 µl of an aqueous solution containing 5 mg/mL insulin (manufactured by TAKARA BIO Inc.) was set as a positive control, and a group with addition of dimethyl sulfoxide was set as a negative control. Thereafter, the medium and the sample were exchanged in the same manner as in the method described in Example 3. The amount of triglyceride in the adipocytes was determined 7 days after addition of the sample.

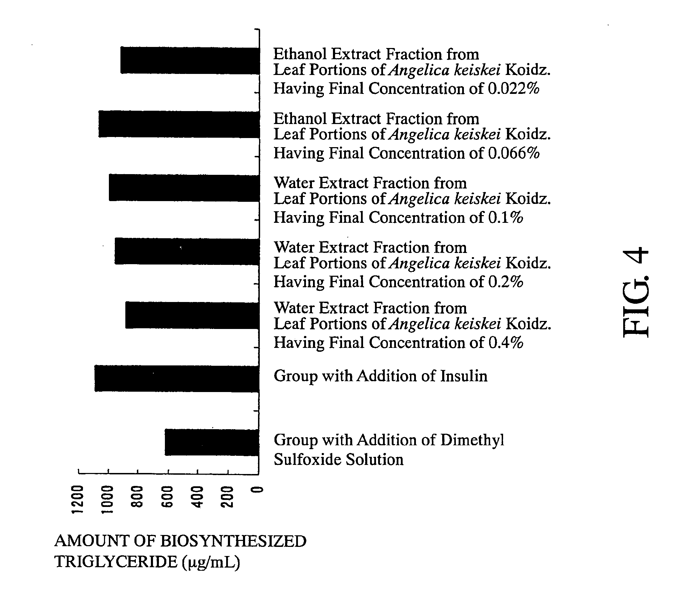

As a result, the induction of triglyceride biosynthesis was found in the group with addition of the water extract fraction from leaf portions of *Angelica keiskei* koidz. and the ethanol extract fraction from leaf portions of *Angelica keiskei* koidz. at each concentration. In other words, the water extract fraction from leaf portions of *Angelica keiskei* koidz. and the ethanol extract fraction from leaf portions of *Angelica keiskei* koidz. were found to have the actions of induction of differentiation into matured adipocytes. The results are shown in Figure 4. In Figure 4, the axis of abscissas is each sample, and the axis of ordinates is the amount of biosynthesized triglyceride (µg/mL).

### Example 8 Preparation of Extract Fraction from Leaf Portions of Apium

Forty milliliters of distilled water, ethanol or ethyl acetate was added to each 2 g of dry powder of leaf portions of *Apium,* and extracted for 30 minutes. The mixture was separated into the extract and the residue by centrifugation. Next, the procedure of extracting the residue with 30 mL of each solvent was repeated twice. The extraction with water was carried out at 60°C, and the extraction with ethanol and the extraction with ethyl acetate were carried out at room temperature. The resulting extracts were collected, and the combined extract was concentrated with a rotary evaporator. Finally, the water extract was dissolved in 10 mL of distilled water, to give a water extract fraction from leaf portions of *Apium.* The ethanol extract was dissolved in 5 mL of dimethyl sulfoxide, to give an ethanol extract fraction from leaf portions of *Apium.* The ethyl acetate extract was dissolved in 5 mL of dimethyl sulfoxide, to give an ethyl acetate extract fraction from leaf portions of *Apium.*

### Example 9 Induction of Adipocyte Differentiation by Extract Fraction from Leaf Portions of Apium

The action of induction of differentiation (insulin-mimetic activity) of the water extract fraction from leaf portions of *Apium,* the ethanol extract fraction from leaf portions of *Apium* and the ethyl acetate extract fraction from leaf portions of *Apium* prepared in Example 8 into matured adipocytes was determined in the same manner as in the method described in Example 3.

Specifically, as a sample, there was set a group with addition of an aqueous solution of the water extract fraction from leaf portions of *Apium* to each well to have a final concentration of 0.1% each, or a group with addition of a dimethyl sulfoxide solution of the ethanol extract fraction from leaf portions of *Apium* or a dimethyl sulfoxide solution of the ethyl acetate extract fraction from leaf portions of *Apium* to each well to have a final concentration of 0.2%, 0.066%, or 0.022%, respectively. A group with addition of 4 µl of an aqueous solution containing 5 mg/mL insulin (manufactured by TAKARA BIO Inc.) was set as a positive control, and a group with addition of dimethyl sulfoxide was set as a negative control. Thereafter, the medium and the sample were exchanged in the same manner as in the method described in Example 3, and the amount of triglyceride in the adipocytes was determined 7 days after addition of the sample.

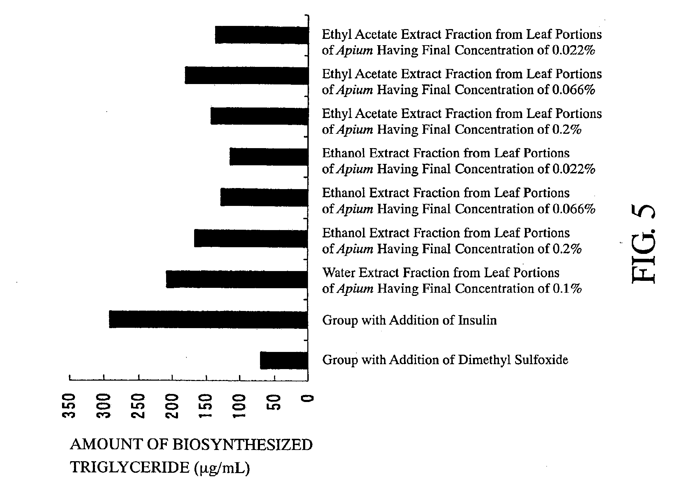

As a result, the induction of triglyceride biosynthesis was found in the group with addition of the water extract fraction from leaf portions of *Apium,* the group with addition of the ethanol extract fraction from leaf portions of *Apium* and the group with addition of the ethyl acetate extract fraction from leaf portions of *Apium* at each concentration. In other words, the water extract fraction from leaf portions of *Apium,* the ethanol extract fraction from leaf portions of *Apium,* and the ethyl acetate extract fraction from leaf portions of *Apium* were found to have the actions of induction of differentiation into matured adipocytes. The results are shown in Figure 5. In Figure 5, the axis of abscissas is each sample, and the axis of ordinates is the amount of biosynthesized triglyceride (µg/mL).

### Example 10 Enhancing Action for Glucose Uptake by Extract Fraction from Leaf Portions of Apium

As evaluation of the enhancing action for glucose uptake of the ethanol extract fraction from leaf portions of *Apium* and the ethyl acetate extract fraction from leaf portions of *Apium* prepared in Example 8, and also as evaluation of the insulin-mimetic action, the amount of 2-deoxyglucose uptake into the matured adipocytes during the stimulation of the sample in the adipocytes was determined in the same manner as in the method described in Example 5.

Specifically, a dimethyl sulfoxide solution of the ethanol extract fraction from leaf portions of *Apium* or a dimethyl sulfoxide solution of the ethyl acetate extract fraction from leaf portions of *Apium* was put in each well as a sample to have a final concentration of 0.2%, or 0.066%, respectively. Here, there were set a group without addition of the sample as a negative control, and a group with addition of insulin so as to have a final concentration of 1 µg/mL as a positive control. Thereafter, the amount of 2-deoxy-[1,2-³H(N)]-glucose uptake into the cells was determined in the same manner.

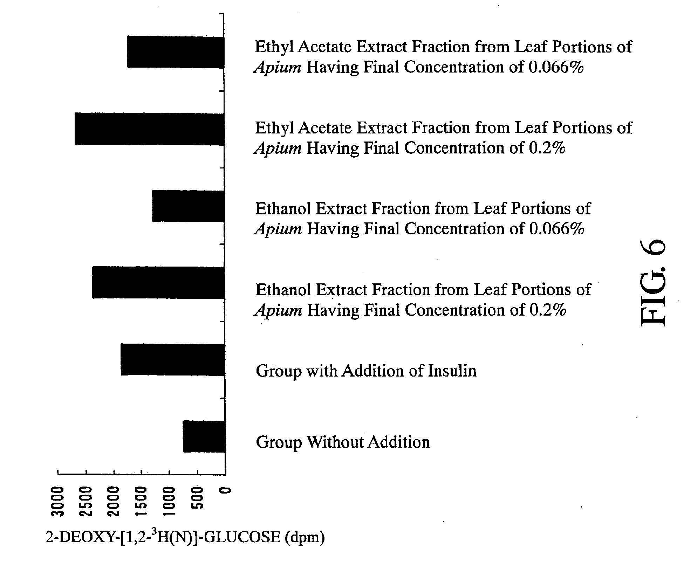

As a result, the enhancement of 2-deoxy[1,2-³H(N)]-glucose uptake was found in the group with addition of the ethanol extract fraction from leaf portions of *Apium* and the ethyl acetate extract fraction from leaf portions of *Apium* at each concentration, as compared to the negative control, in the same manner as that in the group with addition of insulin. In other words, the ethanol extract fraction from leaf portions of *Apium* and the ethyl acetate extract fraction from leaf portions of *Apium* were found to have enhancing activities for glucose uptake. The results are shown in Figure 6. In Figure 6, the axis of abscissas is each sample, and the axis of ordinates is the amount of 2-deoxy[1,2-³H(N)]-glucose (dpm).

### Example 11 Preparation of Extract Fraction from Petroselium sativum

Forty milliliters of distilled water, ethanol or ethyl acetate was added to each 2 g of dry powder of *Petroselium sativum*, and extracted for 30 minutes. Thereafter, the mixture was separated into the extract and the residue by centrifugation. Next, the procedure of extracting the residue with 30 mL of each solvent was repeated twice. The extraction with water was carried out at 60°C, and the extraction with ethanol and the extraction with ethyl acetate were carried out at room temperature. The resulting extracts were collected, and the combined extract was concentrated with a rotary evaporator. Finally, the water extract was dissolved in 10 mL of distilled water, to give a water extract fraction from *Petroselium sativum*. The ethanol extract was dissolved in 5 mL of dimethyl sulfoxide, to give an ethanol extract fraction from *Petroselium sativum*. The ethyl acetate extract was dissolved in 5 mL of dimethyl sulfoxide, to give an ethyl acetate extract fraction from *Petroselium sativum*.

### Example 12 Induction of Adipocyte Differentiation by Extract Fraction from Petroselium sativum

The action of induction of differentiation (insulin-mimetic activity) of the water extract fraction from *Petroselium sativum* prepared in Example 11 into matured adipocytes was determined in the same manner as in the method described in Example 3.

Specifically, as a sample, there was set a group with addition of an aqueous solution of the water extract fraction from *Petroselium sativum* to each well to have a final concentration of 0.4%, 0.2%, or 0.1%, respectively. Here, there were set a group with addition of 4 µl of an aqueous solution containing 5 mg/mL insulin (manufactured by TAKARA BIO Inc.) as a positive control, and a group with addition of dimethyl sulfoxide as a negative control. Thereafter, the medium and the sample were exchanged in the same manner as in the method described in Example 3, and the amount of triglyceride in the adipocytes was determined 7 days after addition of the sample.

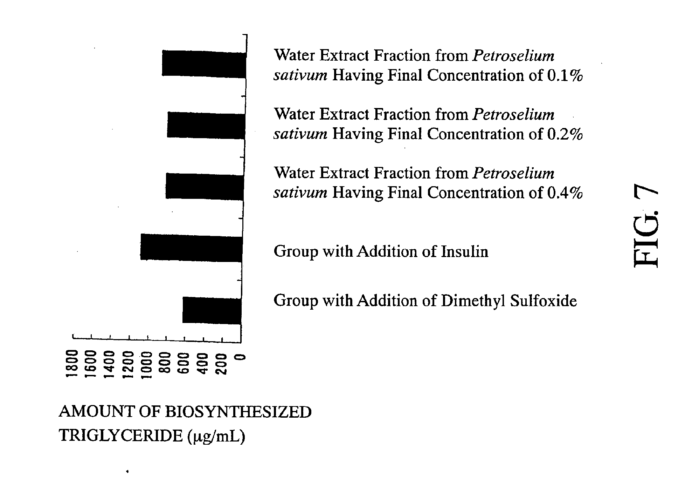

As a result, the induction of triglyceride biosynthesis was found in the group with addition of the water extract fraction from *Petroselium sativum* at each concentration. In other words, the water extract fraction from *Petroselium sativum* was found to have the action of induction of differentiation into matured adipocytes. The results are shown in Figure 7. In Figure 7, the axis of abscissas is each sample, and the axis of ordinates is the amount of biosynthesized triglyceride (µg/mL).

### Example 13 Enhancing Action for Glucose Uptake by Extract Fraction from Petroselium sativum

As evaluation of the enhancing action for glucose uptake of the ethanol extract fraction from *Petroselium sativum* and the ethyl acetate extract fraction from *Petroselium sativum* prepared in Example 11, and also as evaluation of the insulin-mimetic action, the amount of 2-deoxyglucose uptake into the matured adipocytes during the stimulation of the sample in the adipocytes was determined in the same manner as in the method described in Example 5.

Specifically, as a sample, a dimethyl sulfoxide solution of the ethanol extract fraction from *Petroselium sativum* or a dimethyl sulfoxide solution of the ethyl acetate extract fraction from *Petroselium sativum* was put in each well to have a final concentration of 0.2% or 0.066%, respectively. Here, there were set a group without addition of the sample as a negative control, and a group with addition of insulin so as to have a final concentration of 1 µg/mL as a positive control. Thereafter, the amount of 2-deoxy-[1,2-³H(N)]-glucose uptake into the cells was determined in the same manner.

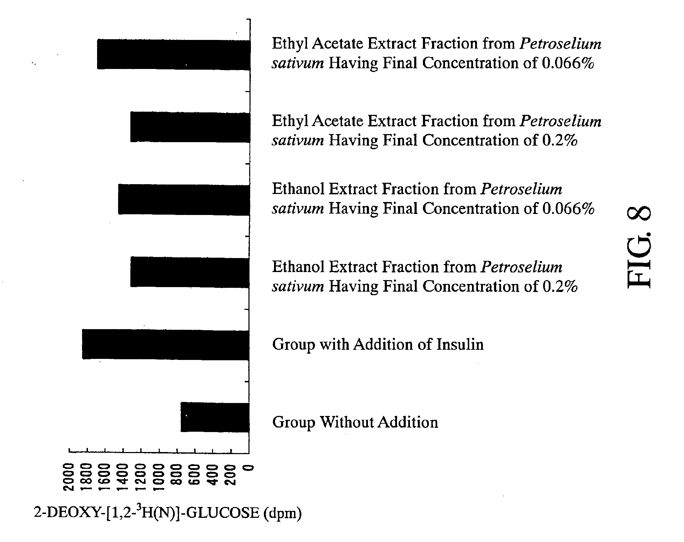

As a result, the enhancement of 2-deoxy[1,2-³H(N)]-glucose uptake was found in the group with addition of the ethanol extract fraction from *Petroselium sativum* and the group with addition of ethyl acetate extract fraction from *Petroselium sativum* at each concentration, as compared to the negative control, in the same manner as that in the group with addition of insulin. In other words, the ethanol extract fraction from *Petroselium sativum* and the ethyl acetate extract fraction from *Petroselium sativum* were found to have enhancing activities for glucose uptake. The results are shown in Figure 8. In Figure 8, the axis of abscissas is each sample, and the axis of ordinates is the amount of 2-deoxy[1,2-³H(N)]-glucose (dpm).

### Example 14 Preparation of Ethanol Extract Fraction and Ethyl Acetate Extract Fraction from Root Portions of Angelica keiskei Koidz.

Forty milliliters of ethanol or ethyl acetate was added to each 2 g of dry powder of root portions of *Angelica keiskei* koidz., and extracted at room temperature for 30 minutes. Thereafter, the mixture was separated into the extract and the residue by centrifugation. Next, the procedure of extracting the residue with 30 mL of each solvent was repeated twice. The resulting extracts were collected, and the combined extract was concentrated with a rotary evaporator. Finally, the ethanol extract was dissolved in 1 mL of dimethyl sulfoxide, to give an ethanol extract fraction from root portions of *Angelica keiskei* koidz. The ethyl acetate extract was dissolved in 1 mL of dimethyl sulfoxide, to give an ethyl acetate extract fraction from root portions of *Angelica keiskei* koidz.

### Example 15 Induction of Adipocyte Differentiation by Ethanol Extract

### Fraction and Ethyl Acetate Extract Fraction from Root Portions of Angelica keiskei Koidz.

The action of induction of differentiation (insulin-mimetic activity) of the ethanol extract fraction from root portions of *Angelica keiskei* koidz. and the ethyl acetate extract fraction from root portions of *Angelica keiskei* koidz. prepared in Example 14 into matured adipocytes was determined in the same manner as in the method described in Example 3.

Specifically, as a sample, there was set a group with addition of a dimethyl sulfoxide solution of the ethanol extract fraction from root portions of *Angelica keiskei* koidz. or a group with addition of a dimethyl sulfoxide solution of the ethyl acetate extract fraction from root portions of *Angelica keiskei* koidz. to each well to have a final concentration of 0.05 or 0.025%, respectively. Here, there were set a group with addition of 4 µl of an aqueous solution containing 5 mg/mL insulin (manufactured by TAKARA BIO Inc.) as a positive control, and a group with addition of dimethyl sulfoxide as a negative control. Thereafter, the medium and the sample were exchanged in the same manner as in the method described in Example 3, and the amount of triglyceride in the adipocytes was determined 7 days after addition of the sample.

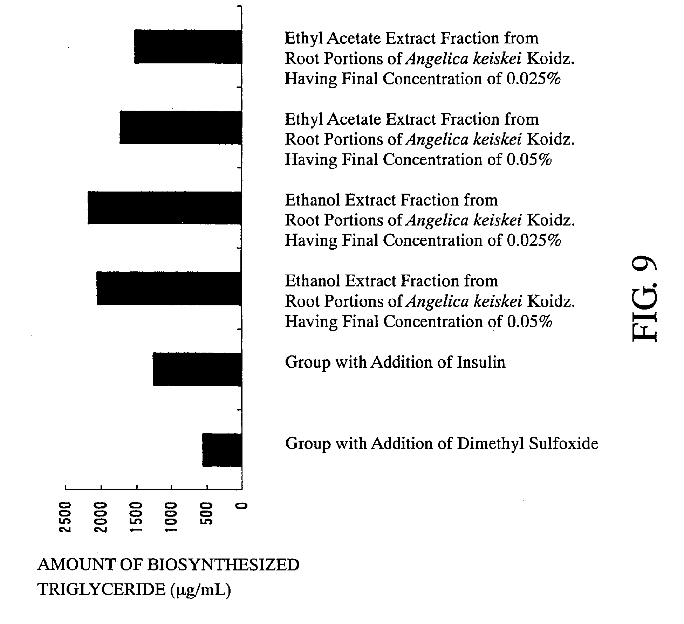

As a result, the induction of triglyceride biosynthesis was found in the group with addition of the ethanol extract fraction from root portions of *Angelica keiskei* koidz. and the group with addition of the ethyl acetate extract fraction from root portions of *Angelica keiskei* koidz. at each concentration. In other words, the ethanol extract fraction from root portions of *Angelica keiskei* koidz. and the ethyl acetate extract fraction from root portions of *Angelica keiskei* koidz. were found to have the actions of induction of differentiation into matured adipocytes. The results are shown in Figure 9. In Figure 9, the axis of abscissas is each sample, and the axis of ordinates is the amount of biosynthesized triglyceride (µg/mL).

### Example 16 Enhancing Action for Glucose Uptake by Ethanol Extract Fraction and Ethyl Acetate Extract Fraction from Root Portions of Angelica keiskei Koidz.

As evaluation of the enhancing action for glucose uptake of the ethanol extract fraction from root portions of *Angelica keiskei* koidz. and the ethyl acetate extract fraction from root portions of *Angelica keiskei* koidz. prepared in Example 14, and also as evaluation of the insulin-mimetic action, the amount of 2-deoxyglucose uptake into matured adipocytes during the stimulation of the sample in the adipocytes was determined in the same manner as in the method described in Example 5.

Specifically, a dimethyl sulfoxide solution of the ethanol extract fraction from root portions of *Angelica keiskei* koidz. or a dimethyl sulfoxide solution of the ethyl acetate extract fraction from root portions of *Angelica keiskei* koidz. was put to each well to have a final concentration of 0.1% or 0.05% as a sample, respectively. Here, there were set a group without addition of sample as a negative control, and a group with addition of insulin so as to have a final concentration of 1 µg/mL as a positive control. Thereafter, the amount of 2-deoxy-[1,2-³H(N)]-glucose uptake into the cells was determined in the same manner.

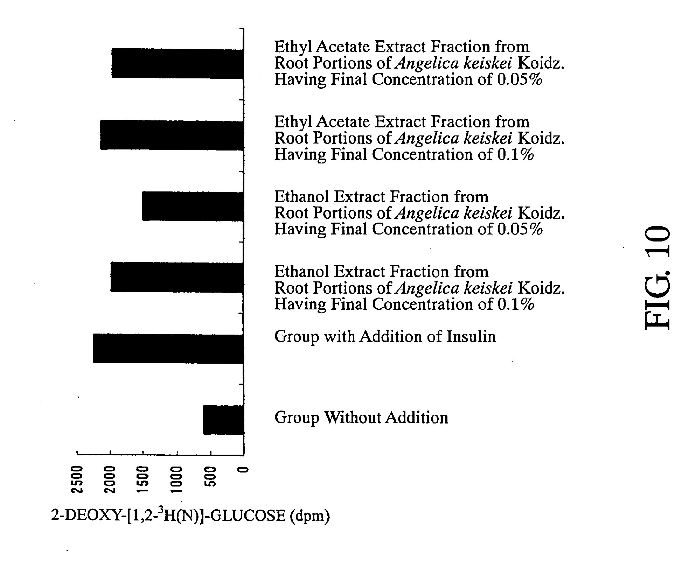

As a result, the enhancement of 2-deoxy[1,2-³H(N)]-glucose uptake was found in the group with addition of the ethanol extract fraction from root portions of *Angelica keiskei* koidz. and the group with addition of ethyl acetate extract fraction from root portions of *Angelica keiskei* koidz. at each concentration, as compared to the negative control, in the same manner as in the group with addition of insulin. In other words, the ethanol extract fraction from root portions of *Angelica keiskei* koidz. and the ethyl acetate extract fraction from root portions of *Angelica keiskei* koidz. were found to have the enhancing activity for glucose uptake. The results are shown in Figure 10. In Figure 10, the axis of abscissas is each sample, and the axis of ordinates is the amount of 2-deoxy[1,2-³H(N)]-glucose (dpm).

### Example 17 Enhancing Action for Glucose Uptake by Extract Fraction from Leaf Portions of Angelica keiskei Koidz.

As evaluation of enhancing action for glucose uptake of the ethanol extract fraction from leaf portions of *Angelica keiskei* koidz. and the ethyl acetate extract fraction from leaf portions of *Angelica keiskei* koidz. prepared in Example 6, and also as evaluation of insulin-mimetic action, the amount of 2-deoxyglucose-uptake into matured adipocytes during the stimulation of the sample in the adipocytes was determined in the same manner as in the method described in Example 5.

Specifically, as a sample, a dimethyl sulfoxide solution of the ethanol extract fraction from leaf portions of *Angelica keiskei* koidz. or a dimethyl sulfoxide solution of the ethyl acetate extract fraction from leaf portions of *Angelica keiskei* koidz. was put in each well to have a final concentration of 0.2% or 0.1%, respectively. Here, there were set a group without addition of the sample as a negative control, and a group with addition of insulin so as to have a final concentration of 1 µg/mL as a positive control. Thereafter, the amount of 2-deoxy-[1,2-³H(N)]-glucose uptake into the cells was determined in the same manner.

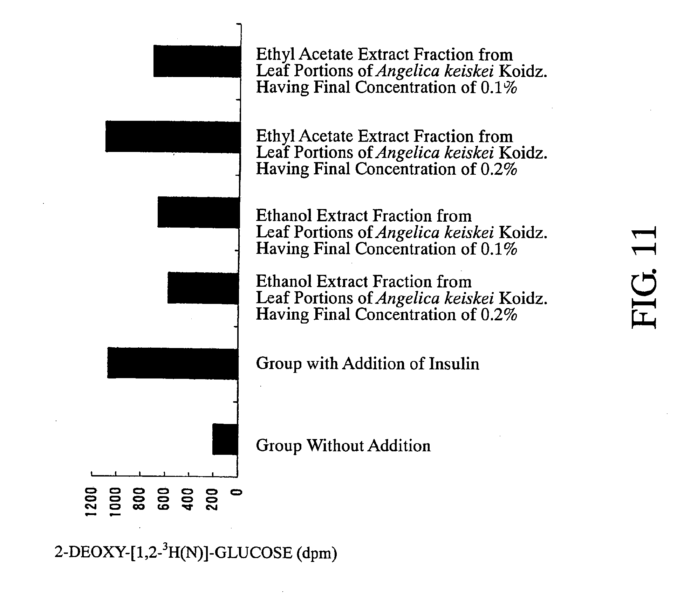

As a result, the enhancement of 2-deoxy[1,2-³H(N)]-glucose uptake was found in the group with addition of the ethanol extract fraction from leaf portions of *Angelica keiskei* koidz. and the group with addition of ethyl acetate extract fraction from leaf portions of *Angelica keiskei* koidz. at each concentration, as compared to the negative control, in the same manner as that in the group with addition of insulin. In other words, the ethanol extract fraction from leaf portions of *Angelica keiskei* koidz. and the ethyl acetate extract fraction from leaf portions of *Angelica keiskei* koidz. were found to show the enhancing activity for glucose uptake. The results are shown in Figure 11. In Figure 11, the axis of abscissas is each sample, and the axis of ordinates is the amount of 2-deoxy[1,2-³H(N)]-glucose (dpm).

### Example 18 Enhancing Action for Glucose Uptake by Fractionated Fraction of Extracts from Root Portions of Angelica keiskei Koidz.

As evaluation of enhancing action for glucose uptake of each fraction of the extract fractions from root portions of *Angelica keiskei* koidz. prepared in Example 2, and also as evaluation of insulin-mimetic action, the amount of 2-deoxyglucose uptake into matured adipocytes during the stimulation of the sample into the cells was determined in the same manner as in the method described in Example 5.

Specifically, as a sample, the silica column-fractionated fraction 1 having a final concentration of 0.03 mg/mL, the silica column-fractionated fraction 2 having a final concentration of 0.013 mg/mL, the silica column-fractionated fraction 3 having a final concentration of 0.0077 mg/mL or the silica column-fractionated fraction 4 having a final concentration of 0.0096 mg/mL was used. Here, there were set a group without addition of the sample as a negative control, and a group with addition of insulin so as to have a final concentration of 1 µg/mL as a positive control. Thereafter, the amount of 2-deoxy-[1,2-³H(N)]-glucose uptake into the cells was determined in the same manner.

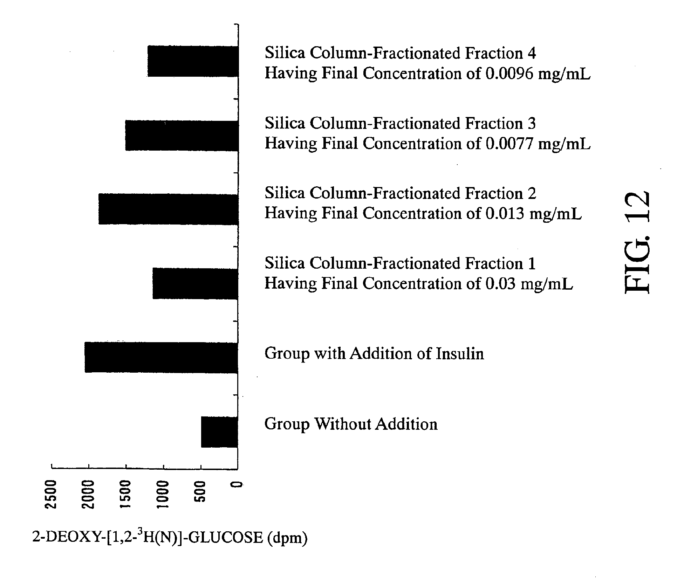

As a result, the enhancement of 2-deoxy[1,2-³H(N)]-glucose uptake was found in the group with addition of each fraction of extract fraction from root portions of *Angelica keiskei* koidz., as compared to the negative control, in the same manner as in the group with addition of insulin. In other words, all of the silica column-fractionated fractions 1, 2, 3 and 4 of the extract fractions from root portions of *Angelica keiskei* koidz. were found to show the enhancing activities for glucose uptake. The results are shown in Figure 12. In Figure 12, the axis of abscissas is each sample, and the axis of ordinates is the amount of 2-deoxy[1,2-³H(N)]-glucose (dpm).

### Example 19 Inhibition of Enhancing Action for Glucose Uptake of Ethanol Extract Fraction from Root Portions of Angelica keiskei Koidz. by Cytochalasin B

The influence of cytochalasin B on 2-deoxyglucose uptake into matured adipocytes during the stimulation of the sample into the cells was tested in the same manner as in the method described in Example 5 on whether the enhancing action for glucose uptake of the ethanol extract fraction from root portions of *Angelica keiskei* koidz. shown in Example 16 is inhibited by cytochalasin B, which is an inhibitor of glucose transporter.

Specifically, as a sample, a group with addition of a dimethyl sulfoxide solution of the ethanol extract fraction from root portions of *Angelica keiskei* koidz. so as to have a final concentration of 0.1% was set. Here, there were set a group without addition of the sample as a negative control, and a group with addition of insulin so as to have a final concentration of 1 µg/mL as a positive control. Furthermore, a group with addition of cytochalasin B (manufactured by nacalai tesque, 10435-81) so as to have a final concentration of 40 µM was set concurrently to the timing of the setting of the group with addition of insulin in each group. Thereafter, the amount of 2-deoxy-[1,2-³H(N)]-glucose uptake into the cells was determined in the same manner.

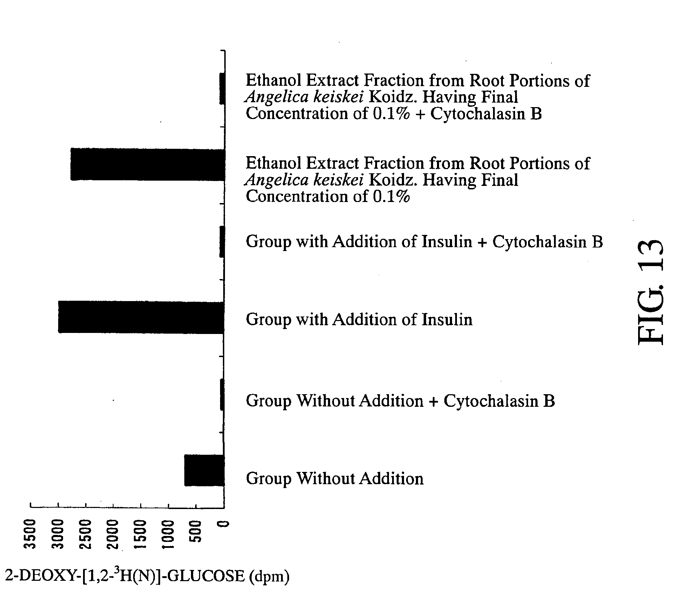

As a result, the enhancement of 2-deoxy-[1,2-³H(N)]-glucose uptake was found in the group with addition of the ethanol extract fraction from root portions of *Angelica keiskei* koidz., as compared to the negative control, in the same manner as in the group with addition of insulin, but the 2-deoxy-[1,2-³H(N)]-glucose uptake was almost completely inhibited by addition of cytochalasin B concurrently with each group. In other words, it could be confirmed that the enhancing activity for glucose uptake by the ethanol extract fraction from *Angelica keiskei* koidz. is mediated by glucose transporter in the same manner as that of insulin. The results are shown in Figure 13. In Figure 13, the axis of abscissas is each sample, and the axis of ordinates is the amount of 2-deoxy-[1,2-³H(N)]-glucose (dpm).

### Example 20 Synergistic Enhancing Action of Glucose Uptake by Ethanol Extract Fraction from Root Portions of Angelica keiskei Koidz. and Insulin

As evaluation of the synergistic enhancing action of glucose uptake by the ethanol extract fraction from root portions of *Angelica keiskei* koidz. prepared in Example 14 and a low concentration-insulin, the amount of 2-deoxyglucose uptake into matured adipocytes during the stimulation of the sample in the adipocytes was determined partly in the same manner as in the method described in Example 5,.

The matured adipocytes were prepared in the same manner as in the method described in item (1) of Example 5.

After the termination of the culture, the medium was removed, and the cells were washed twice with 0.1% (w/v) bovine serum albumin (manufactured by Sigma, A8022)-containing Dulbecco's modified Eagle's medium. Thereafter, 1 mL of the same medium containing a dimethyl sulfoxide solution of the ethanol extract fraction from root portions of *Angelica keiskei* koidz. having a final concentration of 0.02% was added thereto. The cells were cultured overnight at 37°C in the presence of 5% carbon dioxide gas. A group without containing the ethanol extract fraction from root portions of *Angelica keiskei* koidz. was set as a negative control. After the overnight culture, the cells were washed twice with HEPES buffer (140 mM NaCl, 5 mM KCl, 2.5 mM MgSO₄, 1 mM CaCl₂, 20 mM HEPES-Na (pH 7.4)), and 0.9 mL of the same buffer containing the ethanol extract fraction from root portions of *Angelica keiskei* koidz. having a final concentration of 0.02% was added thereto. The cells were cultured at 37°C for 45 minutes. Subsequently, insulin was added thereto so as to have a final concentration of 0.001 µg/mL, and the cells were cultured for additional 30 minutes. At this time, as a control, there was set a group with addition of insulin so as to have a final concentration of 0.001 µg/mL in the wells to which the ethanol extract fraction from *Angelica keiskei* koidz had not been added. In addition, a group without addition of sample was set as a negative control. Thereafter, the amount of 2-deoxy-[1,2-³H(N)]-glucose uptake into the cells was determined in the same manner as in the method described in item (2) of Example 5.

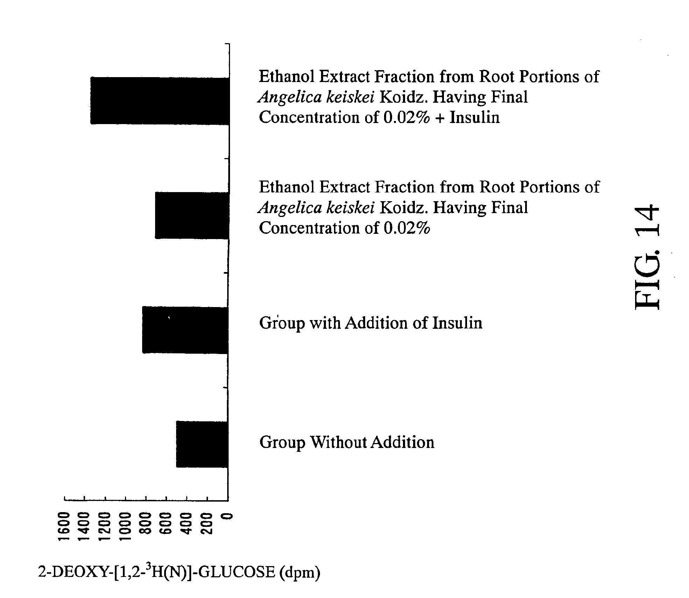

As a result, the enhancement of 2-deoxy-[1,2-³H(N)]-glucose uptake was found in the group with addition of the ethanol extract fraction from root portions of *Angelica keiskei* koidz., as compared to the negative control, in the same manner as in the group with addition of insulin. However, the enhancement of glucose uptake was found in the group with concurrent addition of insulin more than in any of the group with addition of insulin alone and the group with addition of the ethanol extract fraction from root portions of *Angelica keiskei* koidz. alone. In other words, the ethanol extract fraction from root portions of *Angelica keiskei* koidz. was found to have the action for synergistically increasing the enhancing activity for glucose uptake by the concurrent addition of insulin. The results are shown in Figure 14. In Figure 14, the axis of abscissas is each sample, and the axis of ordinates is the amount of 2-deoxy-[1,2-³H(N)]-glucose (dpm).

### Example 21 Enhancement of Glucose Uptake by Insulin Stimulation into Adipocytes Which Were Induced to Be Differentiated by Extract Fraction from Root Portions of Angelica keiskei Koidz.

Adipocytes which were induced to be differentiated by the ethanol extract fraction from *Angelica keiskei* koidz. prepared in Example 14 were obtained, and thereafter it was confirmed whether the glucose uptake into the cells was enhanced by insulin stimulation.

Specifically, as a sample, there was set a group with addition of a dimethyl sulfoxide solution of the ethanol extract fraction from root portions of *Angelica keiskei* koidz. to have a final concentration of 0.05%. A group with addition of 4 µl of an aqueous solution containing 5 mg/mL insulin (manufactured by TAKARA BIO INC.) was set as a positive control. Thereafter, the medium and the sample were exchanged in the same manner as in the method described in Example 3 except that 3-isobutyl-1-methylated xanthine was added to have a final concentration of 0.5 mM concurrently to the timing of the addition of dexamethasone, to give matured adipocytes which were induced to be differentiated by the ethanol extract fraction from root portions of *Angelica keiskei* koidz. or insulin.

Next, the amount of 2-deoxyglucose uptake into the resulting matured adipocytes during the insulin stimulation in the adipocytes was determined in the same manner as in the method described in Example 5.

Specifically, a group without addition of insulin or a group with addition of insulin so as to have a final concentration of 1 µg/mL was set in each of the matured adipocytes. Thereafter, the amount of 2-deoxy-[1,2-³H(N)]-glucose uptake into the cells was determined in the same manner.

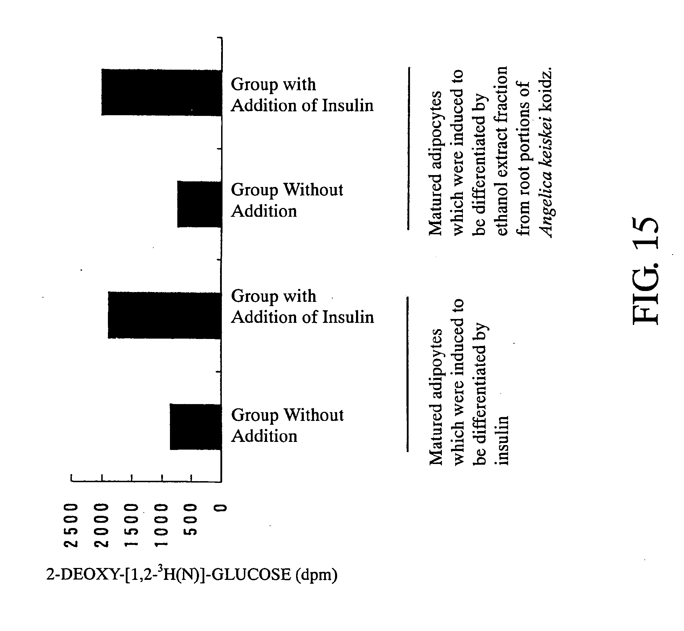

As a result, the enhancement of 2-deoxy[1,2-³H(N)]-glucose uptake was found by insulin stimulation in the matured adipocytes which were induced to be differentiated by the ethanol extract fraction from root portions of *Angelica keiskei* koidz., in the same manner as in the matured adipocytes which were induced to be differentiated by insulin. In other words, the matured adipocytes which were induced to be differentiated by the ethanol extract fraction from root portions of *Angelica keiskei* koidz. were found to show the enhancement of glucose uptake by insulin stimulation. The results are shown in Figure 15. In Figure 15, the axis of abscissas is each sample, and the axis of ordinates is the amount of 2-deoxy[1,2-³H(N)]-glucose (dpm).

### Example 22 Preparation of Extract Fraction from Stem and Leaf Portions of Angelica keiskei Koidz.

Forty milliliters of ethanol was added to 2 g of dry powder of stem and leaf portions of *Angelica keiskei* koidz., and extracted at room temperature for 30 minutes. Thereafter, the mixture was separated into the extract and the residue by centrifugation. Next, the procedure of extracting the residue with 30 mL of the same solvent was repeated twice. The resulting extracts were collected, and the combined extract was concentrated with a rotary evaporator. Finally, the concentrate was dissolved in 1 ml of dimethyl sulfoxide, to give an ethanol extract fraction from stem and leaf portions *of Angelica keiskei* koidz.

### Example 23 Induction of Adipocyte Differentiation by Extract Fraction from Stem and Leaf Portions of Angelica keiskei Koidz.

The action of induction of differentiation into matured adipocytes (insulin-mimetic activity) of the ethanol extract fraction from stem and leaf portions of *Angelica keiskei* koidz. prepared in Example 22 was assayed in the same manner as in the method of Example 3.

Specifically, as a sample, there was set a group with addition of a dimethyl sulfoxide solution of the ethanol extract fraction from stem and leaf portions of *Angelica keiskei* koidz. to each well to have a final concentration of 0.2, 0.066, or 0.022%, respectively. Here, there were set a group with addition of 4 µl of an aqueous solution containing 5 mg/mL insulin (manufactured by TAKARA BIO INC.) as a positive control, and a group with addition of dimethyl sulfoxide as a negative control. Thereafter, the medium and the sample were exchanged in the same manner as in the method described in Example 3, and the amount of biosynthesized triglyceride was determined 7 days after addition of the sample.

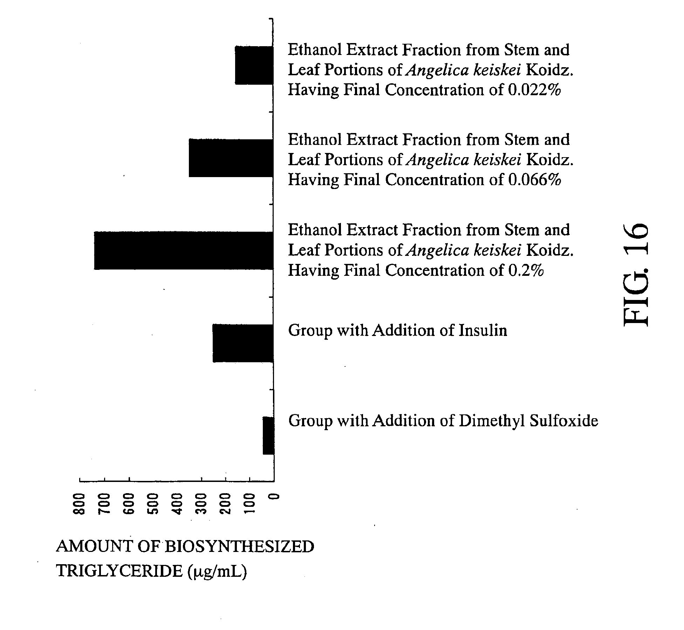

As a result, the induction of triglyceride biosynthesis was found in the group with addition of the ethanol extract fraction from stem and leaf portions of *Angelica keiskei* koidz. at each concentration. In other words, the ethanol extract fraction from stem and leaf portions of *Angelica keiskei* koidz. was found to show the action of induction of differentiation into matured adipocytes. The results are shown in Figure 16. In Figure 16, the axis of abscissas is each sample, and the axis of ordinates is the amount of biosynthesized triglyceride (µg/mL).

### Example 24 Preparation of Ethanol Extract Fraction from Root Portions of Angelica keiskei Koidz.

One liter of ethanol was added to 200 g of dry powder of root portions *Angelica keiskei* koidz., and extracted at room temperature for 30 minutes. After suction filtration, the mixture was separated into the ethanol extract and the residue. Next, the procedure of extracting the residue with 1 liter of the same solvent was repeated once. The resulting ethanol extracts were collected, and the combined extract was concentrated with a rotary evaporator. The resulting concentrate was dissolved in 100 ml of olive oil, to give an ethanol extract fraction from root portions of *Angelica keiskei* koidz.

### Example 25 Preparation of Sample Derived from Yellow Sap of Angelica keiskei Koidz.

Stem portions of *Angelica keiskei* koidz. were cut, and yellow sap was collected from the cross section. The obtained sap was filtered, and the filtrate was lyophilized, to give 2 g of dry powder. Olive oil was added thereto to make up a volume of 10 ml, to give a sample derived from yellow sap of *Angelica keiskei* koidz.

### Example 26 Amelioration Effect on Diabetes of Processed Product from Angelica keiskei Koidz.

Pathological amelioration effects of the ethanol extract fraction from root portions of *Angelica keiskei* koidz. obtained in Example 24 and the sample derived from the yellow sap of *Angelica keiskei* koidz. obtained in Example 25 were studied using type II diabetes model mice. Experiments were carried out with five 11-week-old female KK-Ay mice (manufactured by CLEA Japan, Inc.) per group. Each kind of the samples suspended or dissolved in olive oil was forcibly orally administered to each mouse at 5 mL/kg once a day for consecutive days. Olive oil was administered to each mouse in the control group at 5 mL/kg in the same manner. On the day before the beginning of administration and on the seventh day, blood was collected from the veins of mouse tails, and the blood glucose level was determined with a simple blood glucose meter ACCU-CHEK Compact (manufactured by Roche Diagnostics K.K.). The blood glucose level was found to be remarkably lowered by the administration of the ethanol extract fraction from root portions of *Angelica keiskei* koidz. and the sample derived from yellow sap of *Angelica keiskei* koidz. In addition, no changes were found in body weight and general symptoms during the experimental period.

### INDUSTRIAL APPLICABILITY

According to the present invention, there is provided a medicament, food, beverage, or feed for treating or preventing a disease accompanying an abnormality in an amount of insulin or insulin response, comprising as an effective ingredient a processed product derived from a plant belonging to Umbelliferae. The medicament is useful as a therapeutic agent or prophylactic agent for a disease accompanying an abnormality in an amount of insulin or insulin response, such as diabetes or obesity. Also, the food or beverage can ameliorate the symptoms for a disease accompanying an abnormality in an amount of insulin or insulin response by taking as daily foodstuff. Therefore, the functional foodstuff containing a processed product derived from the plant belonging to Umbelliferae are functional foodstuff useful for maintaining homeostasis of a living body because of their insulin-mimetic action. In addition, according to the present invention, there is provided an agent for insulin-mimetic action, comprising a processed product derived from a plant belonging to Umbelliferae, and the agent for insulin-mimetic action is useful for studies on the function of insulin and screening for a medicament for a disease associated with insulin. In addition, according to the present invention, there is provided an agent for enhancement of glucose uptake into a cell comprising a processed product derived from a plant belonging to Umbelliferae, and the agent for enhancement of glucose uptake is useful for treatment or prevention of a disease requiring the enhancing action for glucose uptake into the cell for the treatment or prevention, manufacture of a food, beverage or feed for treating or preventing the disease, and screening for a drug against a disease requiring the enhancing action for glucose uptake. In addition, according to the present invention, there is provided an agent of induction of an adipocyte differentiation comprising a processed product derived from a plant belonging to Umbelliferae, and the agent of induction of differentiation is useful for the treatment or prevention of a disease requiring the action of induction of an adipocyte differentiation for the treatment or prevention, manufacture of a food, beverage or feed for treating or preventing the disease, and screening for a drug against a disease requiring the action of induction of differentiation.

## Claims

1. A therapeutic agent or prophylactic agent for a disease accompanying an abnormality in an amount of insulin or insulin response, **characterized in that** the agent comprises as an effective ingredient a processed product derived from a plant belonging to Umbelliferae.

2. The therapeutic agent or prophylactic agent according to claim 1, wherein the plant belonging to Umbelliferae is *Angelica keiskei koidz., Apium* or *Petroselium sativum*.

3. An insulin-mimetic action agent, **characterized in that** the agent comprises as an effective ingredient a processed product derived from a plant belonging to Umbelliferae.

4. The agent according to claim 3, wherein the plant belonging to Umbelliferae is *Angelica keiskei koidz., Apium* or *Petroselium sativum*.

5. A food, beverage, or feed for treating or preventing a disease accompanying an abnormality in an amount of insulin or insulin response, **characterized in that** the agent comprises as an effective ingredient a processed product derived from a plant belonging to Umbelliferae.

6. The food, beverage, or feed according to claim 5, wherein the plant belonging to Umbelliferae is *Angelica keiskei* koidz., *Apium* or *Petroselium* *sativum*.

7. An agent for enhancement of glucose uptake into a cell, **characterized in that** the agent comprises as an effective ingredient a processed product derived from a plant belonging to Umbelliferae.

8. The agent according to claim 7, wherein the plant belonging to Umbelliferae is *Angelica keiskei koidz., Apium* or *Petroselium sativum*.

9. An agent for induction of an adipocyte differentiation, **characterized in that** the agent comprises as an effective ingredient a processed product derived from a plant belonging to Umbelliferae.

10. The agent according to claim 9, wherein the plant belonging to Umbelliferae is *Angelica keiskei koidz., Apium* or *Petroselium sativum*.
